# EUROPEAN PATENT SPECIFICATION

(11) **EP 2 391 730 B1**
(45) Date of publication and mention of the grant of the patent: **13.04.2016**
(21) Application number: 10702168.5
(22) Date of filing: 29.01.2010
(51) Int. Cl.: C12Q 1/68

(54) **PREDICTIVE USE OF CPG METHYLATION**
VERWENDUNG VON CPG-METHYLIERUNG ZUR VORHERSAGE
UTILISATION PRÉDICTIVE DE LA MÉTHYLATION CPG

(30) Priority: 30.01.2009 GB 0901534; 13.11.2009 GB 0919910
(43) Date of publication of application: 07.12.2011
(73) Proprietor: University of Southampton, Southampton, Hampshire SO17 IBJ (GB); Auckland Uniservices Limited, Auckland 1142 (NZ); Agresearch Limited, Hamilton 3240 (NZ)
(72) Inventor: GODFREY, Keith Malcolm, Southampton Hampshire SO16 6YD (GB); HANSON, Mark Adrian, Southampton Hampshire SO16 6YD (GB); BURDGE, Graham Charles, Southampton Hampshire SO16 6YD (GB); LILLYCROP, Karen Ann, Southampton Hampshire SO16 6YD (GB); SLATER-JEFFERIES, Joanne Lesley, Southampton Hampshire SO16 6YD (GB); COOPER, Cyrus, Southampton Hampshire SO16 6YD (GB); GLUCKMAN, Peter David, Grafton Auckland 1023 (NZ); SHEPPARD, Allan Michael, Grafton Auckland 1023 (NZ)
(74) Representative: Lord, Hilton David
(86) International application number: PCT/GB2010/050141
(87) International publication number: WO 2010/086663

(56) References cited:
- WO-A1-2007/015993
- WO-A2-2007/129113
- WO-A2-2007/132167
- WO-A2-2008/073303
- WU Q ET AL: "Parental obesity and overweight affect the body-fat accumulation in the offspring: The possible effect of a high-fat diet through epigenetic inheritance" OBESITY REVIEWS, BLACKWELL SCIENCE, GB, vol. 7, no. 2, 21 April 2006 (2006-04-21), pages 201-208, XP002458510 ISSN: 1467-7881
- LI G ET AL: "Identification and characterization of the human retinoid X receptor alpha gene promoter" GENE, ELSEVIER, AMSTERDAM, NL, vol. 372, 10 May 2006 (2006-05-10), pages 118-127, XP024934431 ISSN: 0378-1119 [retrieved on 2006-05-10]
- SMITH F M ET AL: "Regulation of growth and metabolism by imprinted genes" CYTOGENETIC AND GENOME RESEARCH, vol. 113, no. 1-4, 2006, pages 279-291, XP008120552 ISSN: 1424-8581
- WATERLAND ROBERT A: "Epigenetic epidemiology of obesity: application of epigenomic technology." NUTRITION REVIEWS AUG 2008, vol. 66 Suppl 1, August 2008 (2008-08), pages S21-S23, XP002574394 ISSN: 1753-4887
- CAMPIÓN J ET AL: "Individuality and epigenetics in obesity." OBESITY REVIEWS : AN OFFICIAL JOURNAL OF THE INTERNATIONAL ASSOCIATION FOR THE STUDY OF OBESITY JUL 2009, vol. 10, no. 4, July 2009 (2009-07), pages 383-392, XP002574393 ISSN: 1467-789X

### SEQUENCE LISTING

<110> University of Southampton
   Auckland Uniservices Limited
   Agresearch Limited
<120> Predictive Use of CpG Methylation
<130> PX208246WO
<150> GB 0901534.8
   <151> 2009-01-30
<150> GB 0919910.0
   <151> 2009-11-13
<160> 37
<170> PatentIn version 3.3
<210> 1
   <211> 24
   <212> DNA
   <213> artificial
<220>
   <223> Primer
<400> 1
   gagtgaggga ggattgtaag tgat 24
<210> 2
   <211> 20
   <212> DNA
   <213> artificial
<220>
   <223> Primer
<400> 2
   attgtaagtg ataaatagga 20
<210> 3
   <211> 24
   <212> DNA
   <213> artificial
<220>
   <223> Primer
<400> 3
   aacttaaaaa attccactct accc 24
<210> 4
   <211> 24
   <212> DNA
   <213> artificial
<220>
   <223> Primer
<400> 4
   ataaaaaatg taagtggata ttga 24
<210> 5
   <211> 20
   <212> DNA
   <213> artificial
<220>
   <223> Primer
<400> 5
   aaatattacc acactatctc 20
<210> 6
   <211> 24
   <212> DNA
   <213> artificial
<220>
   <223> Primer
<400> 6
   cccataaatc aaaacatcaa tttc 24
<210> 7
   <211> 24
   <212> DNA
   <213> artificial
<220>
   <223> Primer
<400> 7
   ggaattttgg tagagggaaa ttag 24
<210> 8
   <211> 20
   <212> DNA
   <213> artificial
<220>
   <223> Primer
<400> 8
   ttcaaatcaa atttcttaat 20
<210> 9
   <211> 24
   <212> DNA
   <213> artificial
<220>
   <223> Primer
<400> 9
   cctatttcca aaatctccct taac 24
<210> 10
   <211> 20
   <212> DNA
   <213> artificial
<220>
   <223> Primer
<400> 10
   tatataccaa accaatactc 20
<210> 11
   <211> 19
   <212> DNA
   <213> artificial
<220>
   <223> Primer
<400> 11
   caaaatctcc cttaactcc 19
<210> 12
   <211> 24
   <212> DNA
   <213> artificial
<220>
   <223> Primer
<400> 12
   gggattattt agtttgtgag tgat 24
<210> 13
   <211> 17
   <212> DNA
   <213> artificial
<220>
   <223> Primer
<400> 13
   ctcctaatct taccccc 17
<210> 14
   <211> 22
   <212> DNA
   <213> artificial
<220>
   <223> Primer
<400> 14
   tatcccaacc taaaatccaaac 22
<210> 15
   <211> 17
   <212> DNA
   <213> artificial
<220>
   <223> Primer
<400> 15
   gggggtaaga ttaggag 17
<210> 16
   <211> 35
   <212> DNA
   <213> artificial
<220>
   <223> Primer
<400> 16
   aggaagagag tttatttttt tatttttgga ggaag 35
<210> 17
   <211> 56
   <212> DNA
   <213> artificial
<220>
   <223> Primer
<400> 17
   cagtaatacg actcactata gggagaaggc tcaaaaattc aaaccccaac ctatta 56
<210> 18
   <211> 35
   <212> DNA
   <213> artificial
<220>
   <223> Primer
<400> 18
   aggaagagag tagtttgaat gaggtttttt aaaaa 35
<210> 19
   <211> 56
   <212> DNA
   <213> artificial
<220>
   <223> Primer
<400> 19
   cagtaatacg actcactata gggagaaggc taaaaaataa ttaccccttc ccactc 56
<210> 20
   <211> 37
   <212> DNA
   <213> artificial
<220>
   <223> Primer
<400> 20
   aggaagagag tgtggaattt ttggattttt tttagtt 37
<210> 21
   <211> 55
   <212> DNA
   <213> artificial
<220>
   <223> Primer
<400> 21
   cagtaatacg actcactata gggagaaggc tttacaaata taaaccacca caccc 55
<210> 22
   <211> 35
   <212> DNA
   <213> artificial
<220>
   <223> Primer
<400> 22
   aggaagagag ttgtggtttt taggttagat aaaaa 35
<210> 23
   <211> 56
   <212> DNA
   <213> artificial
<220>
   <223> Primer
<400> 23
   cagtaatacg actcactata gggagaaggc tataaaaaaa acaaccttct tttcac 56
<210> 24
   <211> 33
   <212> DNA
   <213> artificial
<220>
   <223> Primer
<400> 24
   aggaagagag gtgttgtttt ttgtggtttt tgg 33
<210> 25
   <211> 56
   <212> DNA
   <213> artificial
<220>
   <223> Primer
<400> 25
   cagtaatacg actcactata gggagaaggc ttctaccctt acaacccaat cataac 56
<210> 26
   <211> 29
   <212> DNA
   <213> artificial
<220>
   <223> Primer
<400> 26
   aggaagagag ggagttgtta gggtttttt 29
<210> 27
   <211> 54
   <212> DNA
   <213> artificial
<220>
   <223> Primer
<400> 27
   cagtaatacg actcactata gggagaaggc tccattaacc acctccacct atac 54
<210> 28
   <211> 35
   <212> DNA
   <213> artificial
<220>
   <223> Primer
<400> 28
   aggaagagag tttaataaag tagttttggg agggt 35
<210> 29
   <211> 55
   <212> DNA
   <213> artificial
<220>
   <223> Primer
<400> 29
   cagtaatacg actcactata gggagaaggc tctatctccc cctaaaacca aacac 55
<210> 30
   <211> 36
   <212> DNA
   <213> artificial
<220>
   <223> Primer
<400> 30
   aggaagagag ggttggatgt gagtttagat ttgtag 36
<210> 31
   <211> 56
   <212> DNA
   <213> artificial
<220>
   <223> Primer
<400> 31
   cagtaatacg actcactata gggagaaggc taaaaaaatt ccatcaaaca ataacc 56
<210> 32
   <211> 32
   <212> DNA
   <213> artificial
<220>
   <223> Primer
<400> 32
   aggaagagag tgtttaggaa ggttgggttt gg 32
<210> 33
   <211> 53
   <212> DNA
   <213> artificial
<220>
   <223> Primer
<400> 33
   cagtaatacg actcactata gggagaaggc tcactcctaa aacccctctt caa 53
<210> 34
   <211> 34
   <212> DNA
   <213> artificial
<220>
   <223> Primer
<400> 34
   aggaagagag gggtttagat gaggaggttg gtta 34
<210> 35
   <211> 56
   <212> DNA
   <213> artificial
<220>
   <223> Primer
<400> 35
   cagtaatacg actcactata gggagaaggc tacaaacccc taccactacc aaaata 56
<210> 36
   <211> 37
   <212> DNA
   <213> artificial
<220>
   <223> Primer
<400> 36
   aggaagagag ttttagggag tggattagaa agttttt 37
<210> 37
   <211> 57
   <212> DNA
   <213> artificial
<220>
   <223> Primer
<400> 37
   cagtaatacg actcactata gggagaaggc tcactcccaa catacactaa aacacaa 57

## Description

### Field of the invention

The present invention relates to use of epigenetic markers, more particularly methylation of one or more selected individual CpGs or CpG groups 5' to the coding region of certain genes, especially in perinatal tissue such as umbilical cord, as a means of predicting diverse phenotypic characteristics in an individual in later life. More specifically, it has been found that determination of such CpG/ CpG group methylation in umbilical cord samples of newborns can be correlated with various phenotypic characteristics in infancy and later pre-puberty childhood, including atopic eczema, cognition and neurobehavioural characteristics and important phenotypic indices associated with disease development such as indices of obesity/ adiposity, including total or percentage body fat, and indices of cardiovascular structure and function including left ventricular mass, carotid artery intima-media thickness, systolic and diastolic blood pressure and pulse wave velocity. Thus, the invention has particular clinical utility in providing early prognostic indication of ability to learn and risk of development of allergic, metabolic, cardiovascular or neurobehavioural disease but is not so confined. Methylation status of specific CpGs or CpG groups in healthy newborns, as determined by analysis of DNA extracted from umbilical cord, has been correlated with later development of phenotypic characteristics as diverse as indices of body composition, including in addition to those noted above total or percentage lean body mass and bone mineral content, indices of cardiovascular health, cognitive function, and neuro-behavourial characteristics. Epigenetic profiling relying on determination of methylation status of selected CpGs and /or CpG groups as a predictive tool for future phenotypic characteristics represents a novel concept relying on new insight into the role of epigenetic change in development.

### Background to the invention

The term 'epigenetic' is used to refer to structural changes to genes that do not alter the nucleotide sequence. Of particular relevance is methylation of CpG dinucleotides 5' to the coding region of genes, most notably in gene promoters, and chemical modifications of the chromatin histone core affecting DNA packaging. Hitherto, it has been assumed that CpGs work in islands and that the CpGs that influence gene expression are those located at the transcription factor binding site of certain genes.

Methylation of such CpGs has been of particular interest in relation to cancer study. However, evidence has more recently accumulated that DNA methylation changes provide a link between environmental influences in early life and later development of phenotypic characteristics.

Epidemiological observations previously implicated early development in the etiology of common chronic diseases, including cardiovascular disease, type-2 diabetes, obesity, metabolic syndrome, non-alcoholic steatohepatitis, impaired skeletal growth, osteoporosis, chronic obstructive airways disease including asthma, susceptibility to infection, mental illness and affective disorder, impaired cognition, cancer, impaired renal function, reproductive health and auto-immune disorders. In humans, nutritional constraint before birth was shown to be associated with increased risk of metabolic syndrome and cardiovascular disease (Godfrey & Barker (2001) Public Health Nutrition 4, 611-624). Analogous causal associations between nutritional constraint during pregnancy and later pathophysiology of the offspring have been demonstrated in animal models. The phenomenon may involve adaptations to fetal physiology which predict an unfavourable postnatal environment, such as limited nutrient availability, and so improve survival. However, if nutrients are abundant, the offspring may be less able to adapt to abundant nutrient supply which can ultimately result in disease (Gluckman & Hanson (2004) Science 305, 1733-1736; Gluckman et al. (2008) N. Engl. J. Med. 2008, 359, 61-73). In humans, a period of nutritional constraint during pregnancy can determine the risk of developing obesity in late middle age (Ravelli et al. (1999) Am. J. Clin. Nutr. 70, 811-816).

It was later observed that maternal protein-restriction during rat pregnancy induces an alteration in the methylation of the glucocortioid receptor (GR) promoter associated with altered expression of the receptor in the liver of juvenile offspring (Lillycrop et al. (2005) J. Nutr. 135 1382-1386). However, this observed change in receptor expression in the liver does not express itself as obesity. The invention stems from the subsequent important finding of the inventors that epigenetic changes in themselves are of prognostic value.

The inventors first recognised that degree of gene methylation can be linked to propensity for a phenotypic characteristic as a result of rat studies linking altered methylation of the GR promoter in rat pup adipose tissue with maternal nutritional status and pup body weight on a high fat diet. Subsequent studies reported in Published International Application WO 2007/129113 (University of Southampton & Auckland Uniservices Limited) confirmed that, equally, degree of methylation of specific gene promoters in human perinatal tissue, e.g. umbilical cord, can be linked to a variety of future phenotypic characteristics embracing both clinically relevant outcomes and quality of life attributes, including but not limited to characteristics of body composition/growth (total and /or proportionate body fat mass, total and /or proportionate lean body mass, bone mineral content or density and height), cognitive development (intellectual quotient (IQ)), neuro-behavioural status (e.g. hyperactivity) and cardiovascular structure and function (e.g. blood pressure, aortic compliance, left ventricular mass and coronary artery diameter).

Those findings solely related to degree of epigenetic change associated with gene promoters as a whole. As indicated above, the inventors have now found that detection of methylation status of individual selected CpG dinucleotides or CpG groups and combinations of these 5' to the coding region of certain genes in umbilical cord samples can be correlated with development of phenotypic characteristics in later childhood; more particularly as detailed in the exemplification the inventors firstly looked at correlation between degree of methylation of individual CpGs and CpG groups in umbilical cord and various phenotypic characteristics at 9 years of age. Taking account of sex, methylation status of selected CpGs/ CpG groups was found capable of explaining up to 50% of the variance in body composition and cardiovascular risk markers in normal children with otherwise unremarkable development. These studies lay foundation for a very different approach to predicting future development of phenotypic characteristics of clinical interest, which may be termed 'CpG fingerprinting'.

### Summary of the invention

In its broadest aspect, the present invention thus provides a method of predicting future development of one or more phenotypic characteristics, such as one or more indices of obesity or adiposity selected from such indices including total or proportionate body fat, body mass index and trunk/limb fat ratio as an indicator of body fat distribution in a human, preferably as a newborn, said method comprising
(a) determining in genomic DNA of said human extracted from a tissue sample, e.g. umbilical cord, the methylation status of one or more of the following CpG dinucleotides and /or CpG groups 5' to the coding region of the retinoid X receptor-alpha RXRA gene as identified by genomic coordinates according to the UCSC human genome March 2006 assembly

| |
|---|
| RXRA chr9:136355885+ |
| RXRA chr9:136355556,136355560+ |
| RXRA chr9:136355836+ |

(b) wherein the methylation status of each selected CpG or CpG group correlates with propensity for one or more indices of obesity or adiposity selected from such indices including total or proportionate body fat, body mass index and trunk/ limb fat ratio as an indicator of body fat distribution and
(c) comparing each methylation status determined as in (a) with correlation between methylation status of the relevant CpG or CpG group and variance of one or more indices of obesity or adiposity selected from such indices including total or proportionate body fat, body mass index and trunk/ limb fat ratio as an indicator of body fat distribution in individuals of the same species at a selected older age.

It will be understood that such a prediction method is applied to healthy, normal individuals generally at an early stage in development. Where the sample used for DNA analysis is umbilical cord or another perinatal tissue sample, then in comparing step (b) one or more correlations will generally be relied on obtained with reference to variance of a phenotypic characteristic at a later age in infancy or childhood, e.g. in human children post-infancy and at pre-puberty. For such comparing, it may be found preferable to take account of gender.
(i) Disclosed herein is a method of predicting future development of one or more phenotypic characteristics in a human or non-human animal, preferably as a newborn, wherein said one or more phenotypic characteristics are selected fromone or more indices of obesity or adiposity selected from such indices including total or proportionate body fat, body mass index and trunk/ limb fat ratio as an indicator of body fat distribution; or
(ii) one or more of total lean body mass, proportionate lean mass, bone mineral content and height; or
(iii) one or more indices of cardiovascular structure and function selected from such indices including left ventricular mass, aortic root diameter, coronary artery diameter, carotid artery intima-media thickness, systolic and/or diastolic blood pressure, pulse rate, aorto-foot pulse wave velocity and aorto-femoral pulse wave velocity; or
(iv) one or more indices of cognitive function; or
(v) one or more characteristics of neurobehavioral status selected from such characteristics including, but not limited to, hyperactivity, emotional problems, conduct problems, peer problems, prosocial behaviour or total difficulties; or
(vi) one or more allergic disorders selected from such disorders including, but not limited to, atopic eczema;
said method comprising:
(a) determining in genomic DNA of said human or non-human animal extracted from a tissue sample, e.g. umbilical cord, the methylation status of one or more individual selected CpG dinucleotides and /or CpG groups 5' to the coding region of one or more chosen genes, wherein the methylation status of each selected CpG or CpG group correlates with propensity for one or more relevant phenotypic characteristics, and
(b) comparing each methylation status determined as in (a) with correlation between methylation status of the relevant CpG or CpG group and variance of at least one phenotypic characteristic of interest amongst individuals of the same species at a selected older age.

Useful CpGs or CpG groups for this purpose have surprisingly been found to include such sites both within and outside promoter regions. Furthermore, it has interestingly been found that the methylation status of combinations of CpGs and / or CpG groups 5' to the coding regions of different genes in different mechanistic pathways predict outcomes. Methylation status of more than one CpG/ CpG group may in some instances be relied on 5' proximal to the same gene coding region, e.g. in the same promoter.

Genes of interest on the basis of the studies detailed in the exemplification have been identified as the genes for retinoid X receptor-alpha (RXRA). Although not part of the present invention, also disclosed are genes of interest endothelial nitric oxide synthase (eNOS), superoxide dismutase-1(SOD1), phosphoinositide-3-kinase, catalytic, delta polypeptide (PIK3CD), interleukin-8 (IL-8), peroxisome proliferator-activated receptor gamma 2 (PPARg2) and lipoprotein lipase (LPL). Others may be readily identified in similar manner including for prediction of further phenotypes. It is noteworthy that all the genes noted above are not parentally imprinted genes consistent with the perinatal environment influencing development through epigenetic changes in non-imprinted genes.

By way of example of particular interest, RXRA chr9:136355885+ methylation, e.g. in umbilical cord DNA, is now proposed as an epigenetic marker for propensity for future development of childhood obesity (see Example 1).

Such use of epigenetic markers may be of particular use not only in managing propensity for occurrence of undesirable phenotypic characteristics in humans, e.g. obesity, but also, for example, in enabling economic decisions on future production characteristics in agricultural animals. Such use of epigenetic markers enables the targeting of corrective strategies where propensity for impaired ability to learn or an undesirable phenotypic characteristic is identified.

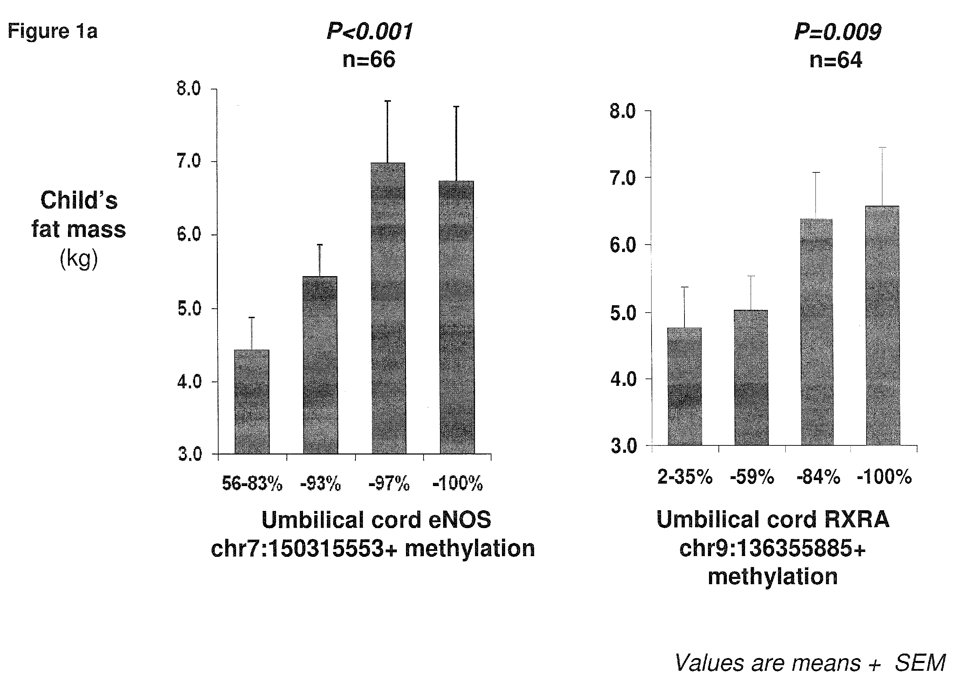

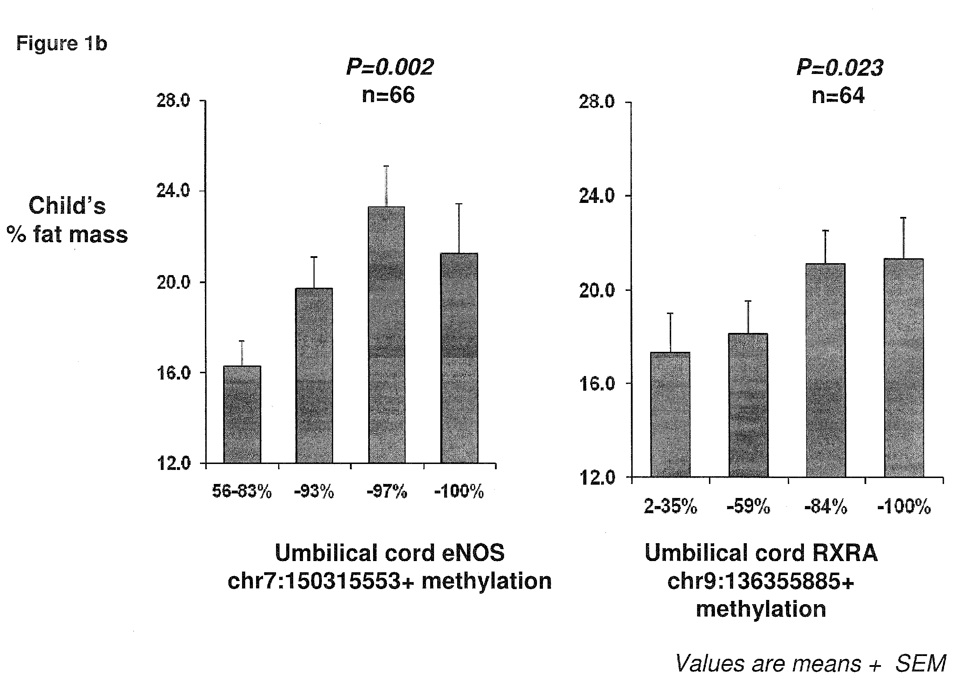

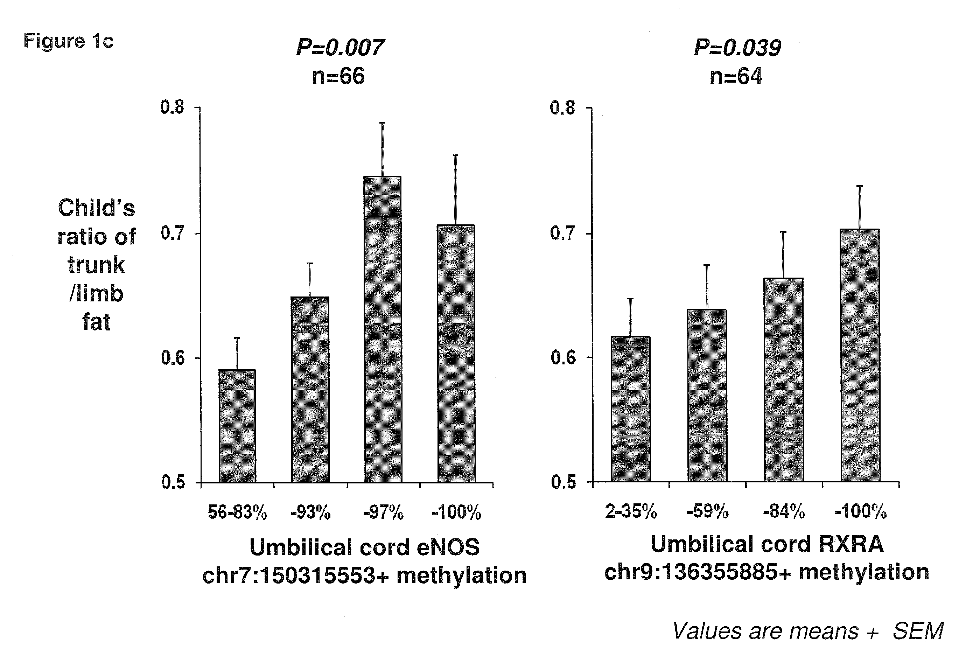

Further aspects of the invention will be apparent from the more detailed description and Example 1, with reference to the figures 1, 7 and 8 below.

### Brief description of the figures

In the figure descriptions below CpGs are identified by the relevant gene, chromosome and genomic coordinate according to the UCSC human genome March 2006 assembly. In all the figures, values are means + SEM.
Figure 1 a shows from the human studies reported in Example 1 positive correlation between percentage methylation in umbilical cord of both RXRA chr9:136355885+ and eNOS chr7:150315553+ and child's fat mass, adjusted for sex, as measured by dual energy X-ray absorptiometry (DXA) at age 9.
Figure 1b shows from the human studies reported in Example 1 positive correlation between percentage methylation in umbilical cord of both RXRA chr9:136355885+ and eNOS chr7:150315553+ and child's percentage fat mass, adjusted for sex, as measured by DXA at age 9.

Figure 1c shows from the human studies reported in Example 1 positive correlation between percentage methylation in umbilical cord of both RXRA chr9:136355885+ and eNOS chr7:150315553+ and child's trunk/ limb fat ratio at age 9, adjusted for sex, as measured by DXA at age 9.

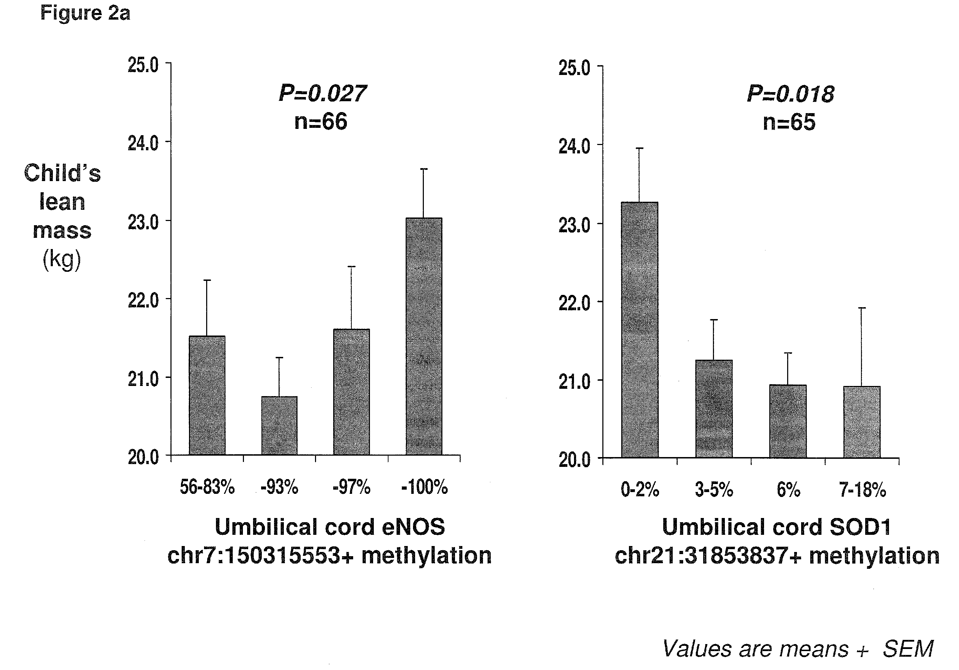

The comparative example in Figure 2a shows from the human studies reported in comparative Example 1 positive correlation between percentage methylation in umbilical cord of eNOS chr7:150315553+ and child's lean mass, adjusted for sex, as measured by DXA at age 9 and inverse correlation between percentage methylation in umbilical cord of SOD1 chr21:31853837+ and child's lean mass determined in the same manner at the same age.

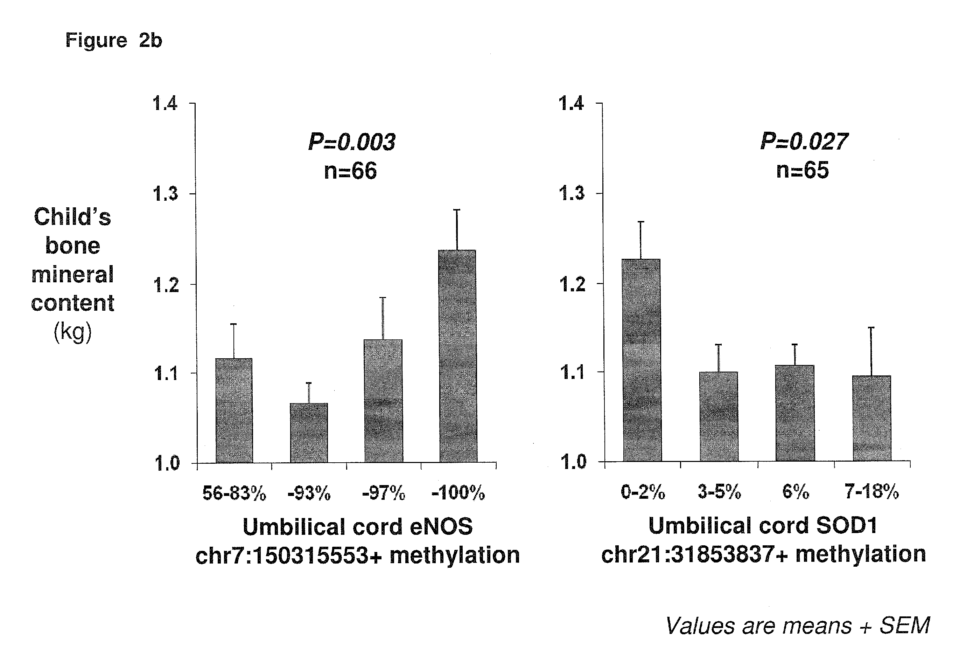

The comparative example in Figure 2b shows from the human studies reported in comparative Example 1 positive correlation between percentage methylation in umbilical cord of eNOS chr7:150315553+ and child's bone mineral content, adjusted for sex, at age 9 and inverse correlation between percentage methylation in umbilical cord of SOD1 chr21:31853837+ and child's bone mineral content, adjusted for sex, at the same age.

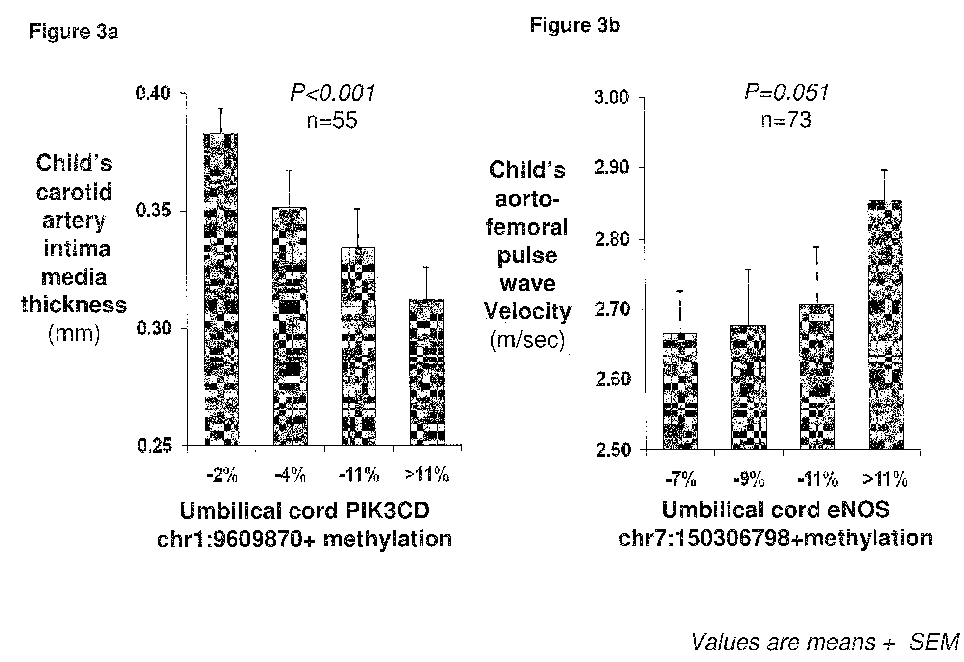

The comparative example in Figure 3a shows from the human studies reported in comparative Example 2 inverse correlation between percentage methylation in umbilical cord of PIK3CD chr1:9609870+ and carotid artery intima media thickness at age 9.

The comparative example in Figure 3b shows from the human studies reported in comparative Example 2 positive correlation between percentage methylation in umbilical cord of eNOS chr7:150306798+ and aorto-femoral pulse wave velocity at age 9.

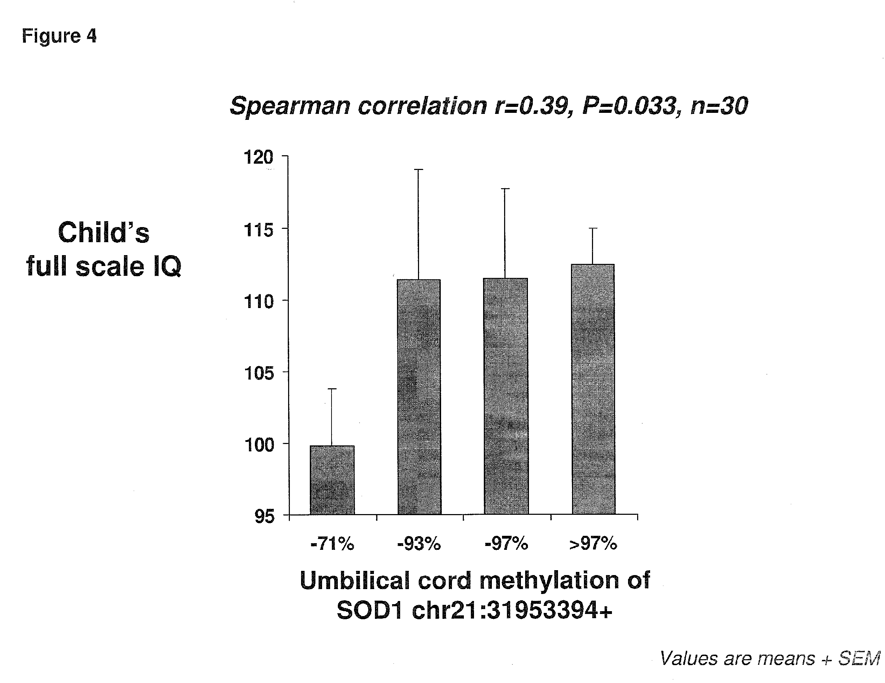

The comparative example in Figure 4 shows from the human studies reported in comparative Example 3 correlation between percentage methylation in umbilical cord of SOD1 chr21:31953394+ and full scale IQ at age 9 (n = 30 children).

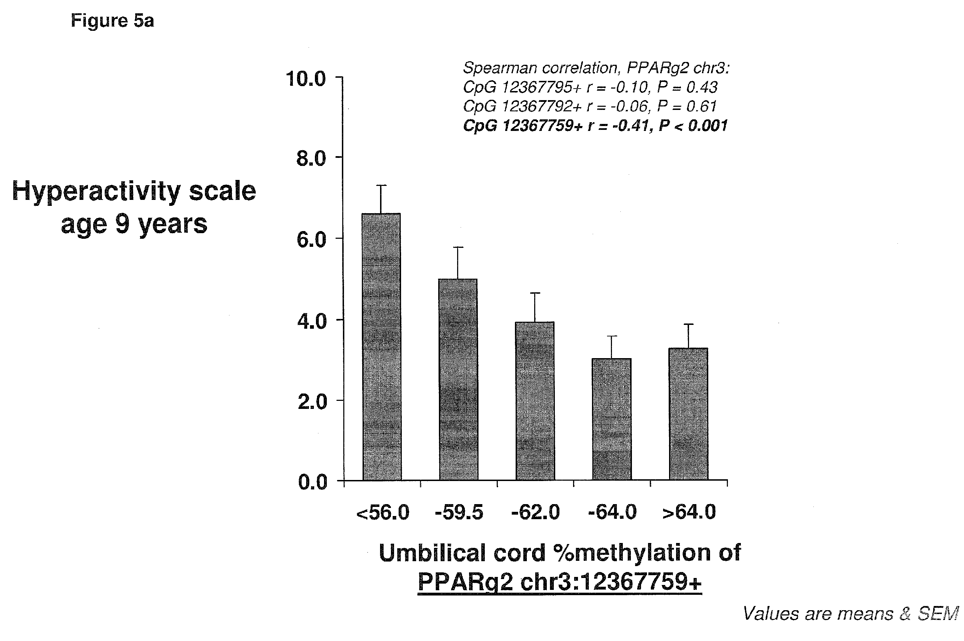

The comparative example in Figure 5a shows from the human studies reported in comparative Example 4 correlation between percentage methylation in umbilical cord of PPARg2 chr3:12367759+ and hyperactivity at age 9 (n = 67).

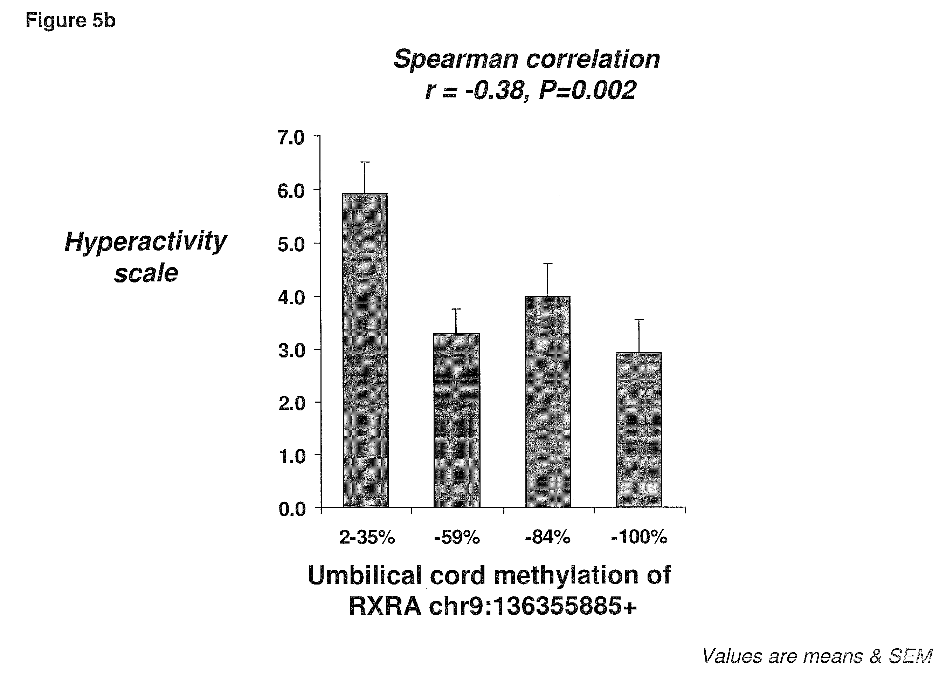

The comparative examples in Figure 5b shows from the human studies reported in Example 4 correlation between percentage methylation in umbilical cord of RXRA chr9:136355885+ and hyperactivity at age 9 (n =63).

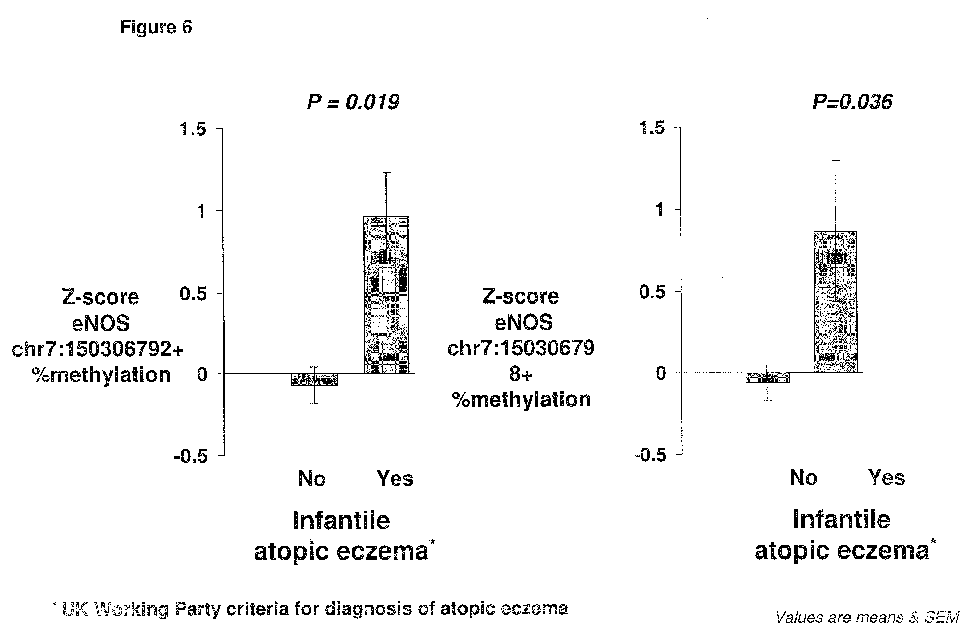

The comparative example in Figure 6 shows association between percentage methylation in umbilical cord of both eNOS chr7:150306792+ and eNOS chr7:150306798+ as determined by pyrosequencing and presence of infantile atopic eczema in infants aged 9 months (n = 74).

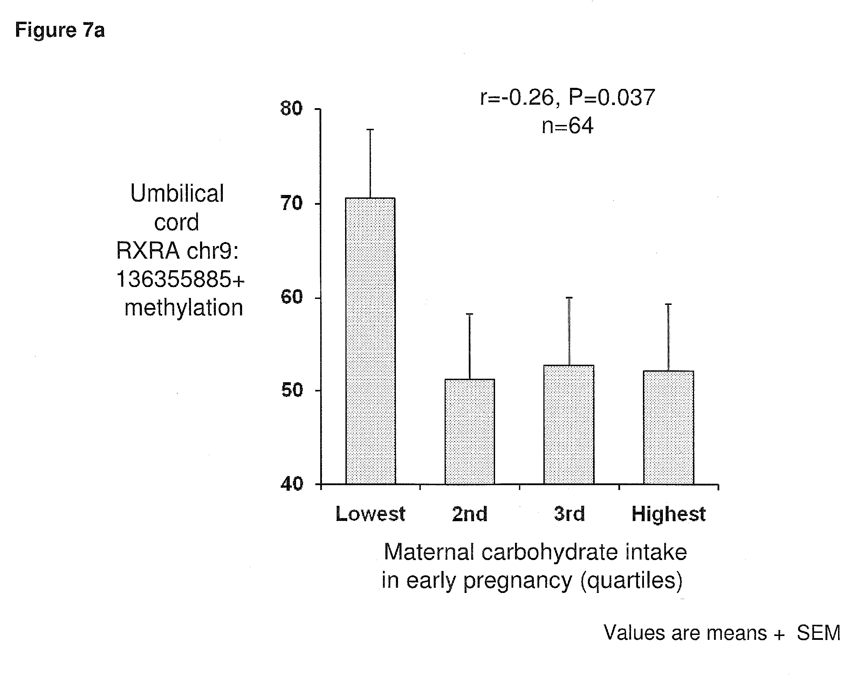

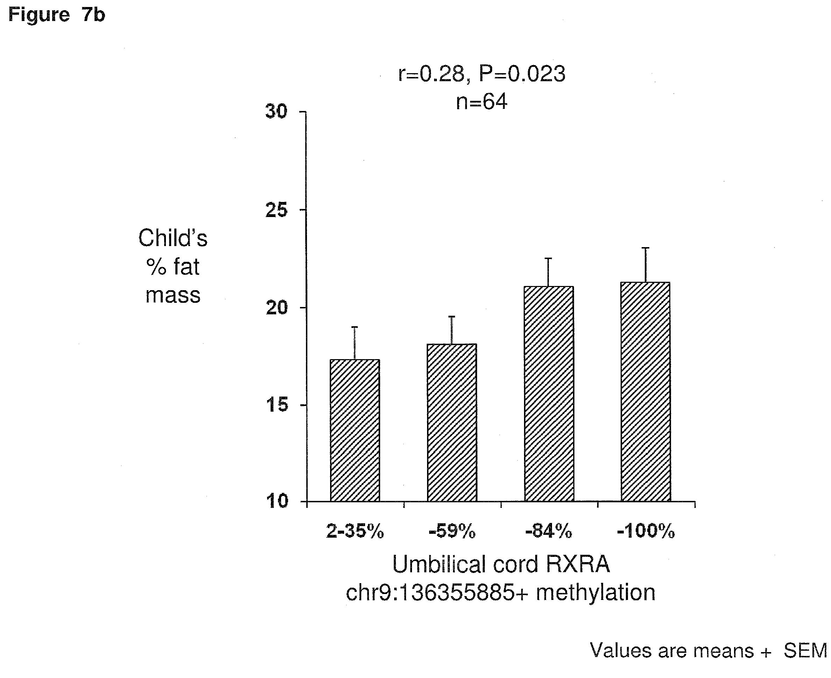

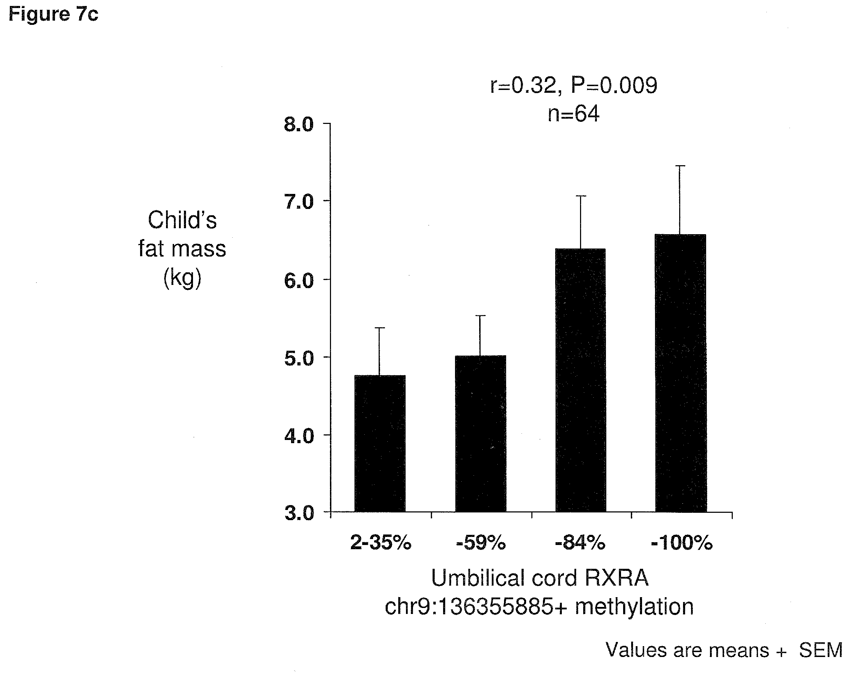

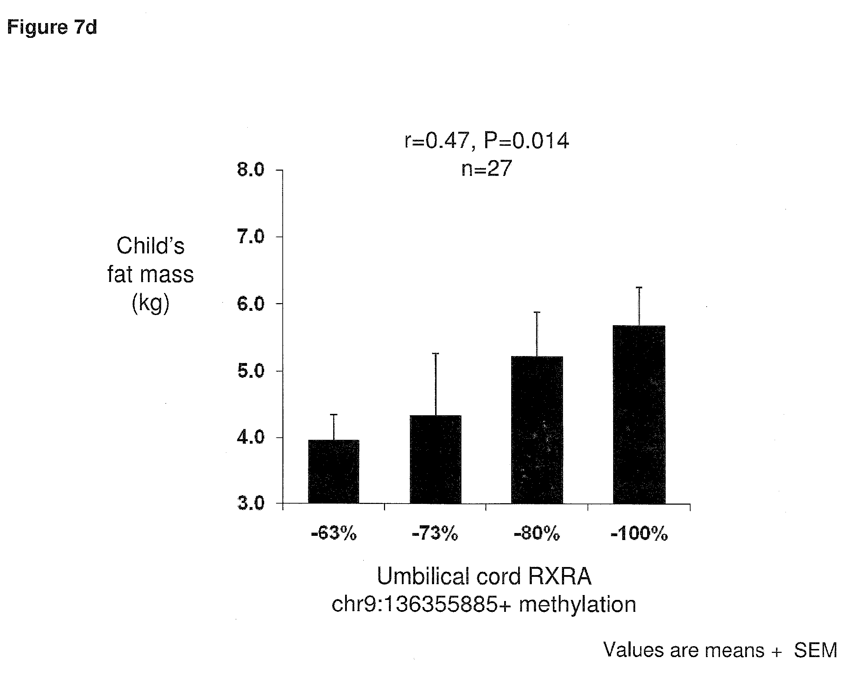

Figure 7: Maternal diet is associated with epigenetic umbilical cord RXRA chr9:136355885+ methylation, and percentage methylation of this CpG site relates to child's later adiposity in two independent cohorts. **(a)** Lower maternal carbohydrate in early pregnancy is associated with higher umbilical cord RXRA chr9:136355885+ methylation in PAH study pregnancies. **(b)** Child's %fat mass at age 9 years increases with higher umbilical cord RXRA chr9:136355885+ methylation in PAH study pregnancies. **(c)** Child's fat mass at age 9 years has a similar positive correlation with RXRA chr9:136355885+ methylation in PAH study pregnancies. **(d)** In a second independent cohort of SWS children, child's adiposity at age 6 years increases with higher umbilical cord RXRA chr9:136355885+ methylation. (PAH=Princess Anne Hospital, SWS=Southampton Women's Survey)

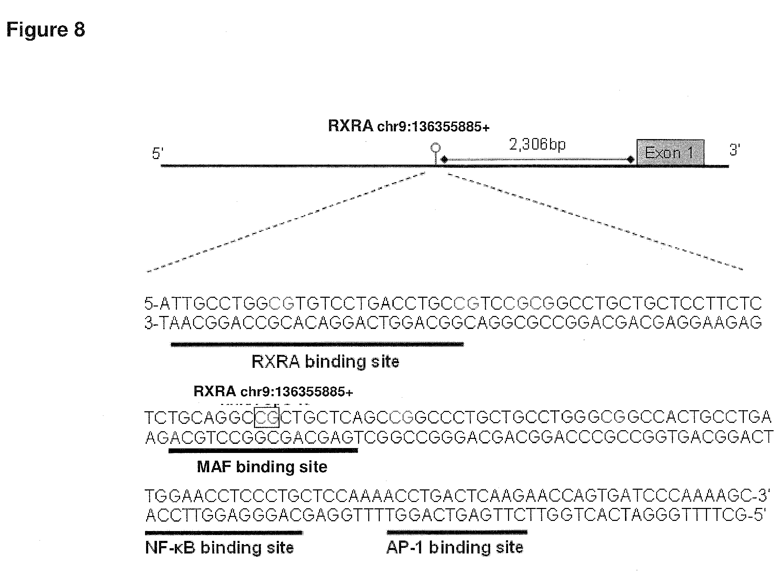

Figure 8: Schematic diagram of the RXRA promoter region, showing the position of the CpG group at RXRA chr9:136355885+ and of neighbouring transcription factor binding sites.

### Detailed description

As indicated above, the tissue used for detection of methylation status of a CpG or CpG group of interest will desirably be a tissue readily available in early life. It may be from a neonate, infant, child or young adult. Desirably, however, it will be a perinatal tissue sample containing genomic DNA of the individual of interest taken prior to, at or soon after birth (generally in the case of human neonates within a month of birth), preferably, for example, an umbilical cord sample, but other tissue may prove useful including adipose tissue, blood (including fetal and cord blood), placenta, chorionic villus biopsy, amniotic fluid, hair follicles, buccal smears and muscle biopsies. Preferably, such tissue will be from a newborn or taken from an infant within a few weeks of birth, more preferably within a few days of birth (less than a week), although samples taken much later, e.g. within 6 months or longer may prove useful.

The selected sites of epigenetic methylation of the invention are the following CpG dinucleotides

| |
|---|
| RXRA chr9:136355885+ |
| RXRA chr9:136355556,136355560+ |
| RXRA chr9:136355836+ |

, for which association has been identified between degree of methylation and variance of a phenotypic characteristic of interest, such as total and /or proportionate body fat mass, in 9 year old children relying on umbilical cord samples as specified in the examples. It will be understood, however, that correlation of methylation status of the CpG or CpG group may be compared with variance of a phenotypic characteristic of interest at any age at which such variance is evident, although generally it will be found desirable to employ in comparison step (b) one or more pre-determined correlations between a methylation marker and variance of a phenotypic characteristic of interest in individuals at an early age, preferably before adulthood. It will be appreciated that a strength of the invention is provision of the ability to predict the likely occurrence of one or more indices of obesity or adiposity selected from such indices including total or proportionate body fat, body mass index and trunk/ limb fat ratio as an indicator of body fat distribution at an early age at which targeted intervention to reduce the identified health risk can have real benefit.

By way of example, as evident from the exemplification, the invention can be used to predict from DNA of a tissue sample taken at an early age, desirably a perinatal tissue sample such as umbilical cord, health risk associated with propensity for any of the following phenotypic characteristics at a later age, preferably while still in childhood and targeted intervention to modify the effect of the relevant phenotype on health is possible:
(a) body composition indices of obesity including total fat mass, proportionate fat mass, trunk/ limb fat ratio (body fat distribution) and body mass index, of interest in identifying individuals at risk of type-2 diabetes, metabolic syndrome, cardiovascular disease and other conditions for which obesity is recognised to be a risk factor (see Example 1, Tables 1-3, and Figures 1 and 7 for illustration of suitable methylation marker sites).

Disclosed herein in the comparative examples, is a method to predict from DNA of a tissue sample taken at an early age, desirably a perinatal tissue sample such as umbilical cord, health risk associated with propensity for any of the following phenotypic characteristics at a later age, preferably while still in childhood and targeted intervention to modify the effect of the relevant phenotype on health is possible:
(b) body composition indices such as total lean body mass, proportionate lean body mass and bone mineral content, and height, of interest in identifying individuals who may be susceptible to, for example, sarcopenia, impaired muscle function, impaired linear growth and disease states linked with low bone mineral content such as osteoporosis in later life (see comparative Example 1, Tables 2 and 4 and Figures 2a and b for illustration of suitable methylation marker sites);
(c) indices of cardiovascular structure and function such as left ventricular mass, aortic root diameter, coronary artery diameter, carotid artery intima-media thickness, systolic and diastolic blood pressure, pulse rate, aorto-foot pulse wave velocity and aorto-femoral pulse wave velocity of interest, for example, in predicting propensity for left ventricular hypertrophy, coronary heart disease, atherosclerosis and hypertension (see comparative Example 2, Tables 5 and 6 and Figure 3 for illustration of suitable methylation marker sites);
(d) indices of neurobehavioural disorders such as emotional disorders, hyperactivity conduct problems, peer problems or total difficulties of interest, for example, in predicting behavioural disorders; evidence is presented of linkage between particular epigenetic methylation markers and cognitive function as indicated by verbal IQ and /or performance IQ and /or full scale IQ score of interest, for example, in predicting impaired ability to learn (see comparative Examples 3 and 4, Tables 7 and 8 and Figures 4 and 5 for illustration of suitable methylation marker sites);
(e) atopic eczema and other allergic disorders (see comparative Example 5, Table 9 and Figure 6 for illustration of suitable methylation marker sites)

As indicated above, in relation to predicting propensity for future development of childhood obesity, assessment of the methylation status of the CpG site RXRA chr9:136355885+ , e.g. in umbilical cord DNA, may, for example, be particularly favoured. This has been shown to positively correlate with both child's fat mass and % fat mass in two independent child cohorts at ages 9 and 6 respectively. Lower maternal carbohydrate in early pregnancy is also shown by data presented herein to be associated with higher umbilical cord RXRA chr9:136355885+ methylation in keeping with proposed use of such methylation as a biomarker for predilection to future obesity (see Figure 7).

Once the propensity for exhibiting phenotypes has been determined in an individual, management of that individual's lifestyle (diet, behaviour, exercise, etc) can be undertaken to reduce the risk of the actual occurrence of any undesirable characteristic such as obesity or low mineral bone content associated with osteoporosis.

Where a method of the invention identifies propensity for obesity in a newborn or child, then this may be used as a cue to also look at the nutritional status of the mother in view of the previously identified link between such propensity and poor maternal nutrition, and where poor maternal nutritional is found, improving the diet of the individual, e.g. by providing dietary advice and /or food supplements.

The methodology of the invention may be used to look at the occurrence of propensity for one or more phenotypic characteristics of the present invention in a chosen population group and thereby used to identify external factors which contribute to the incidence of the characteristic(s) in the population of interest. In this way, information may be obtained useful in directing public health initiatives aimed at reducing the incidence of undesirable phenotypic characteristics in populations and thereby associated disorders such as diabetes, osteoporosis, and cardiac problems. The utility of this invention is that it allows selection of appropriate ways of managing an individual for optimal health and wellbeing.

As indicated above, the inventors previously presented evidence that altered GR promoter methylation in rat adipose tissue is predictive of obesity in late life, but without reference to any individual epigenetic modification of a CpG or CpG group (see Example 1 of WO 2007/129113). Nevertheless, it is reasonable to now extrapolate that, while the illustrations of the invention provided herein relate to humans, methods of the invention can also be applied to animals and are of particular interest in relation to both domestic animals and farm animals. By way of example, early prediction of obesity in farm animals (e.g. cattle, sheep, pigs, chickens, deer) by means of application of the invention would enable farmers to select and / or manage animals so as to maximise production efficiency and thereby economic returns (e.g. carcass yield of lean meat and breed or slaughter decisions). Similarly early diagnosis of predisposition to obesity would enable interventions to manage the condition and maximise animal performance in the bloodstock industries (e.g. horses) and manage obesity in companion animals (e.g. cats and dogs).

Detection of methylation of a CpG or CpG group for the purpose of application of the invention may be achieved by known methods starting with bisulphite treatment of a sample of extracted genomic DNA to convert methylated cytosines to uracil. This may be followed by PCR amplification and sequencing, e.g. pyrosequencing on a Pyromark MD system (Biotage) of bisulphite-treated DNA and an equivalent DNA sample without bisulphite treatment. Alternatively, the same DNA samples may be subjected to Sequenom analysis in which bisulphite treatment is followed by PCR amplification with introduction of a T7 promoter tag, RNA transcription, uracil specific cleavage of transcripts and analysis of the cleavage products by matrix assisted desorption ionization time-of-flight (MALDI-TOF) mass spectrometry (Ehrich et al. (2005) Proc. Natl. Acad. Sci. 102, 15785-90; www.Sequenom.com). In some cases using the latter method, individual CpGs close together will not be resolvable in which case they will be analysed as a group.

In a further aspect, the invention provides a kit for carrying out a method of the invention comprising: (i) primers suitable for obtaining amplicons to analyse the methylation status of one or more selected CpG dinucleotides and /or CpG groups of the invention optionally together with means for bisulphite treatment of DNA and (ii) information on correlation between each said methylation status and variance of at least one phenotypic characteristic of interest.

The examples below illustrate the invention with reference to identification of correlations between the epigenetic state of individual CpGs and/or CpG groups 5' to the coding regions of 8 selected genes in umbilical cord samples and major childhood phenotypes at age 9. Example 1 b extends data linking methylation of certain CpG sites in umbilical cord DNA, e,g RXRA chr9: 136355885+, to childhood adiposity by utilisation of data for a second cohort of children with measured fat mass and %fat mass at age 6. However, as indicated above it is envisaged that the identified methylation markers may be utilised in predicting the same phenotypes at earlier or later ages, except for predicting likelihood of infantile atopic eczema, post-infancy and preferably still in childhood.

### Examples

### Methods

### Samples

Umbilical cord samples were taken from pregnancies in the University of Southampton/UK Medical Research Council Princess Anne Hospital Nutrition Study.

### Subjects and phenotyping

In 1991-2, Caucasian women at least 16 years old with singleton pregnancies of less than 17 weeks' gestation were recruited at the Princess Anne Maternity Hospital in Southampton, UK; diabetics and those who had undergone hormonal treatment to conceive were excluded. In early (15 weeks of gestation) and late (32 weeks of gestation) pregnancy, a dietary and lifestyle questionnaire was administered to the women. Anthropometric data on the child were collected at birth and a section of umbilical cord was collected and stored. Gestational age was estimated from menstrual history and scan data. 559 children were followed-up at age nine months, when research nurses recorded data on anthropometry and infant feeding, and examined the child's skin, looking for evidence of visible atopic eczema. Mothers were also asked whether the child had dry skin or an itchy skin condition since birth and a diagnosis of atopic eczema was defined using a modified version of the UK Working Party's diagnostic criteria for atopic dermatitis (Williams et al., (1994), Br J Dermatol. 131, 406-16). A history of asthma or hay fever was omitted as a criterion as the children were too young to have developed these disorders.

When the children approached age nine years, the parents of those still living in Southampton were invited to permit their children to participate in a further study. Of 461 invited, 216 (47%) agreed to attend a clinic. 87 of these had had an umbilical cord sample collected and stored at - 80°C and were used in the studies reported below. Collection and analysis of umbilical cord samples and follow up of the children was carried out with the written informed consent of all subjects and under Institutional Review Board approval from the Southampton and South West Hampshire Joint Research Ethics Committee.

A second independent cohort of children was also selected for whom umbilical cord data was available and adiposity data at age 6 (for further details see Example 1b and supplementary table S6).

### Assessment of body composition

At age 9 years, height was measured using a stadiometer and weight using digital scales (SECA Model No.835). The children underwent measurements of body composition by DXA (dual energy x-ray absorptiometry; Lunar DPX-L instrument using specific paediatric software; version 4.7c, GE Corporation, Madison, WI, USA). The instrument was calibrated every day and all scans were done with the children wearing light clothing. The short-term and long-term coefficients of variation of the instrument were 0.8% and 1.4% respectively.

### Assessment of cardiac structure, arterial compliance and carotid intima-media thickness

Children attended a clinic at the Princess Anne Hospital, Southampton. At the clinic, they sat quietly in a temperature-controlled room (20±2 °C) for at least 10 mins. When pulse rate and blood pressure (BP) measurements indicated hemodynamic stability, transthoracic echocardiography (Acuson 128 XP and a 3.5MHz phased array transducer) was performed by a single ultrasonographer with the child in the left lateral recumbent position. Two-dimensional, M-mode, and Doppler echocardiograms were recorded over five consecutive cardiac cycles and measurements were made off-line. Aortic root diameter, left ventricular mass (according to American Echocardiography Society convention) and total coronary artery diameter were measured as reported previously (Jiang et al. (2006) Pediatrics 117, e257-61).

While the child was recumbent the ultrasonographer used a 7MHz linear-array transducer to measure intima-media thickness in the right distal portion of the common carotid artery (Gale et al. (2006) Arterioscler. Thromb. Vasc. Biol. 26, 1877-1882). Three longitudinal views, including lateral, antero-posteral and antero-oblique sections, were recorded at the end of the diastolic phase. Intima-media thickness of the far wall was measured 10mm proximal to the beginning of bifurcation, using CVI software (National Instruments; Austin, TX, USA). The mean of the three measurements was used in the analysis.

Arterial compliance was measured by a non-invasive optical method that determines the transit time of the wave of dilatation propagating in the arterial wall, as a result of the pressure wave generated by contraction of the left ventricle. Measurement of the time taken for the wave to travel a known distance allows the velocity of the pulse wave to be calculated. The optical method has been validated against intra-arterial determinations of pressure wave velocity (Bonner et al. (1995) Fortschitt Berichte. 107, 43-52).

Pulse wave velocities were measured in two arterial segments, aorta to femoral, extending from the common carotid artery near the arch of the aorta to the femoral artery just below the inguinal ligament, and aorta to foot, extending to the posterior tibial artery (Gale et al. (2008) Eur. J. Clin. Nutr. 62, 68-77). Pulse wave velocity is inversely related to the square root of the compliance of the vessel wall. High-pulse wave velocity therefore indicates a stiffer arterial wall.

Supplementary Table S1 summarises the body composition characteristics and cardiovascular status of the 87 children at age 9 included in the study.

### Assessment of cognitive function and psychological health

Cognitive function was measured using the Wechsler Abbreviated Scale of Intelligence (WASI) (Wechsler D., Wechsler Abbreviated Scale of Intelligence. The Psychological Corporation, Sam Antonio, 1999) and psychological health was assessed with the Strengths and Difficulties Questionnaire (SDQ), which was completed by the mother (Goodman R. The Strengths and Difficulties Questionnaire: a research note. J. Child Psychol. Psychiatr. (1997) 38:581-586). The SDQ is made up of 5 subscales assessing prosocial behaviour, hyperactivity, emotional symptoms, conduct problems and peer problems.

### Extraction of DNA

Genomic DNA was extracted from 100-200 mg frozen crushed umbilical cord samples (which are comprised primarily of the two umbilical arteries, the umbilical vein and Wharton's jelly).

### Array based analysis

After methylation-specific chromatin precipitation, a commercially available microarray (NimbleGen Human ChIP Epigenetic Promoter Tiling Array) was used to undertake a genome wide assessment of the degree of CpG methylation of putative promoters (Rauch et al. (2007) Proc. NatI. Acad. Sci. USA 104, 5527-32; Weber et al. (2007) Nat. Genet. 39, 457-66). This analyses 5 kb of promoter region for the comprehensive human genome, split across 2 arrays (each of ~12,000 genes), tiling regions at 110 bp intervals and using variable length probes (~5/promoter). Annotated splice variants and alternative splice sites are represented on the array. In consequence of the high cost of the arrays, we therefore undertook measurements on 16 subjects; in one subject, one of the two arrays in this preliminary study failed for technical reasons.

### Quantitative DNA methylation analysis at specifc CpG sites

Genomic DNA was isolated from frozen archived umbilical cord samples by classical proteinase K digestion and phenol: chloroform extraction. Quantitative analysis of DNA methylation at specific CpG sites was carried out using either the Pyromark MD System (Biotage) or the Sequenom MassARRAY Compact System (http://www.sequenom.com).

### Pyrosequencing

Briefly, this involves the gene-specific amplification of bisulfite-treated DNA, followed by sequencing by synthesis through a four-enzyme process using the Pyromark MD. 2µg of DNA was bisulfite converted using the EZ DNA methylation-gold kit (Zymo Research) as per the manufacturer's protocol. PCR primers specific for bisulfite converted DNA were designed using PSQ Assay Design Software. Either the forward or reverse primer included a 5' biotin modification, which is necessary to bind the PCR products to sepharose beads during the pyrosequencing protocol.

Supplementary Table S2 lists amplicons, primer sequences (PCR and pyrosequencing), PCR annealing temperature, and genomic co-ordinates. 1µL of bisulfite-treated DNA was PCR amplified in a 50µL reaction using Qiagen HotStar Taq Polymerase and 10pM final primer concentration as per Pyrosequencing recommendations. PCR conditions consisted of 95°C for 15mins followed by 45 cycles of 94°C for 30 seconds, 50-60°C for 30 seconds and 72°C for 1 min. The final PCR step consisted of a 10min extension at 72°C. No-template controls were included for each amplicon to monitor PCR specificity. Following PCR amplification, the product was annealed to streptavidin-coated sepharose beads and denatured with 0.2M NaOH to a single-stranded product. Following cleaning with Wash Buffer (Biotage) to neutralize the DNA, a pyrosequencing primer was added that is necessary for the initialization of the pyrosequencing reaction. Nucleotides were incorporated to the open 3' DNA strand in which pyrophosphate is released and used in a sulfurylase reaction emitting ATP. The ATP is then used by luciferase, which is converted to oxyluciferin. Light is discharged as a result of the reaction and collected by a CCD camera. The light was assembled into a readable format and represented as peaks. Peak detection, signal-to-noise calculations and quantitative CpG site methylation was performed using Pyro Q-CpG software v1.0.9 (Biotage). Reproducibility data for PPARg2 chr3:12367795+ and PPARg2 chr3:12367792+ showed R² values ranging from 0.78 to 0.90.

### Sequenom analysis

Briefly, as indicated above, this involves the gene-specific amplification of bisulfite-treated DNA, followed by *in vitro* transcription and analysis by matrix-assisted laser desorption ionization time-of-flight (MALDI-TOF) mass spectrometry (Ehrich et al. (2005) Proc. Natl. Axcad. Sci. USA 102, 15785-90). 1µg of DNA was bisulfite converted using EZ DNA Methylation kit (Zymo Research) as per the manufacture's protocol with Sequenom recommendations (alternative cycling protocol, 100µL elution volume). PCR primers specific for bisulfite converted DNA were designed using Methprimer (Li and Dahiya (2002) Bioinformatics 18, 1427-31). Each reverse primer contained a T7-promoter tag for in vitro transcription (5'-cagtaatacgactcactatagggagaaggct-3'), and the forward primer was tagged with a 10mer to balance Tm (5'-aggaagagag-3').

Supplementary Table S3 lists amplicons, primer sequences, PCR annealing temperature, and genomic co-ordinates. 1µL of bisulfite-treated DNA was PCR amplified in a 5µL reaction using Qiagen HotStar Taq Polymerase and 200nM final primer concentration as per Sequenom recommendations. PCR conditions consisted of 94°C for 15mins followed by 45 cycles of 94°C for 20 seconds, 52-62°C for 30 seconds and 72°C for 1 min. The final PCR step consisted of a 3min extension at 72°C. No-template controls were included for each amplicon to monitor PCR specificity. Following PCR amplification, the reaction mixture was treated with Shrimp Alkaline Phosphatase (Sequenom), heat inactivated, and 2µL was used as template in a 7µL simultaneous *in vitro* transcription/ uracil-cleavage reaction as per manufacturer's instructions (Sequenom). Transcription cleavage products were desalted by the addition of 20µL H₂O and 6mg of CLEAN Resin (Sequenom) and spotted on a 384-pad SpectroCHIP (Sequenom) using a MassARRAY nanodispenser (Samsung). Mass spectra were acquired using a MassARRAY MALDI-TOF MS (Bruker-Sequenom) and peak detection, signal-to-noise calculations and quantitative CpG site methylation was performed using proprietary EpiTyper software v1.0 (Sequenom). For fragments containing a single CpG site, DNA methylation state was calculated by the ratio of methylated to unmethylated fragments. For cleavage products containing multiple CpG sites, the proportion of fragments containing one or more methylated site(s) is reported. Non-quantifiable or ambiguous CpG units were excluded from the analysis.

### Statistical analysis

Birth weight was adjusted for gestational age at birth. The epigenetic variables were transformed using a Fisher-Yates transformation to satisfy statistical assumptions of normality, and outcome variables were transformed where necessary using logarithms. Pearson correlation (rₚ) and linear regression were used to examine the relation between epigenetic measurements and child characteristics, adjusting for sex and age at examination where appropriate. The statistical package Stata was used to relate the degree of methylation in umbilical cord to offspring phenotype at age 9.

### Selection of candidate genes

Array analysis was used to select a preliminary panel of genes which showed variation in methylation and strong correlations between methylation status and aspects of later body composition, cardiovascular structure or function at age 9 as described in WO 2007/129113. From this preliminary panel, 8 genes were chosen on the basis of their biological plausibility for measurement of methylation at individual CpGs or CpG groups using pyrosequencing and /or Sequenom analysis.

### Selection of methylation sites for further study

Supplementary tables S4 and S5 show the distributions of CpG methylation at the individual methylation sites analysed, identified with reference to genomic corordinates (UCSC, human genome March 2006 assembly). For the majority of CpGs/ CpG groups studied, methylation was low (75^{th} centile < 5%) and / or there was little variation (difference 5^{th}-95^{th} centiles < 10%), but 37 CpGs/ CpG groups met the criteria of both ≥5% methylation in more than a quarter of the subjects and ≥10% variation in absolute methylation between the 5^{th} and 95^{th} centiles. Particularly marked variation in methylation was seen at some of these sites, including RXRA chr9:136355885+ (median 59%, 5^{th}-95^{th} centiles 4-99%) and eNOS chr7:150315553+ (median 93%, 5^{th}-95^{th} centiles 64-100%). Correlations between methylation of different CpGs were generally low (for example rₚ=0.03, P=0.81, n=55 between the above two CpGs). At the above-mentioned 37 CpGs/CpG groups there are no known single nucleotide polymorphisms. The levels of methylation at those sites in umbilical cord of newborns were therefore compared with phenotypic characteristics of the same individuals at age 9.

### Overview of results

As noted above, strong correlations were identified between the epigenetic state of individual CpGs/CpG groups and combinations of CpGs/CpG groups in umbilical cord tissue and major childhood phenotypes at age 9 years:
- such CpGs/CpG groups and combinations of CpGs/CpG groups can be 5' proximal to the same gene coding region and /or exert their effects through other genes
- such combinations of CpGs/CpG groups may be 5' to the coding regions of genes in different mechanistic pathways
- such CpGs/CpG groups are not limited to the proximal promoter
- phenotypes include phenotypes clinically relevant for health outcomes and quality of life attributes (including body composition/growth, impaired ability to learn, behavioural/cognitive development, cardiovascular structure and function, atopic eczema and allergic disorders)
- perinatal epigenetic methylation markers explain a substantial proportion of variance in phenotypes
- correlations are not unidirectional.

As noted above, data presented in Example Ib importantly indicates that strong positive correlation between methylation status of RXRA chr9:136355885+ and adiposity at age 9 (determined as total fat mass or %fat mass) also applies at age 6

### Example 1 a

### Childhood fat mass, proportionate fat mass, body fat distribution and body mass index at age 9 years

For 19 of the 37 CpGs/CpG groups studied, evidence was found that the degree of methylation was associated at age 9 with the child's total and/or proportionate body fat mass and/or body fat distribution and/or body mass index (see Table 1). Umbilical cord methylation of retinoid X receptor-alpha (RXRA) chr9:136355885+ and endothelial nitric oxide synthase (eNOS) chr7:150315553+ had strong positive associations with child's fat mass (Figure 1 a), percentage fat mass (Figure 1 b), ratio of trunk/limb fat (Figure 1 c) and body mass index (rₚ=0.39, P=0.001 and rₚ=0.26, P=0.037, respectively) at age 9 years. There were large differences in adiposity across the distributions of umbilical cord CpG methylation; for example, mean sex-adjusted fat mass, percentage fat mass and trunk/limb fat ratio rose from 4.8 kg, 17.3% and 0.617, respectively, in the lowest quarter of RXRA chr9:136355885+ to 6.6 kg, 21.3% and 0.704 in the highest quarter of the distribution. Table 2 shows univariate and multivariate analyses of CpG sites for which methylation levels had significant independent associations with the child's adiposity. Taking account of the child's sex, methylation level of RXRA chr9:136355885+ and eNOS chr7:150315553+ explained over 40% of the variances in the child's fat mass and percentage fat mass at age 9 years, and 28% of the variance in body fat distribution. Independently of sex, RXRA chr9:136355885+ and eNOS chr7:150315553+ and superoxide dismutase-1 (SOD1) chr21:31853660/63+ methylation were also inversely related to child's trunk/limb fat ratio (P=0.037). As indicated above, linkage of methylation status of these sites with indicators of obesity is expected to be of assistance in identifying individuals at risk of, for example, type-2 diabetes, metabolic syndrome, cardiovascular disease and other conditions for which obesity is recognised to be a risk factor.

**Table 1: CpGs/CpG groups whose methylation was associated at age 9 with the child's total and/or proportionate body fat mass and/or body fat distribution and/or body mass index identified by genomic coordinates (UCSC, human genome March 2006 assembly)**

| |
|---|
| RXRA chr9:136355885+ |
| RXRA chr9:136355556,136355560+ |
| RXRA chr9:136355593,136355600+ |
| RXRA chr9:136355688+ |
| RXRA chr9:136355836+ |
| RXRA chr9:136357082,136357085,136357087+ |
| RXRA chr9:136357196+ |
| eNOS chr7:150315553+ |
| eNOS chr7:150306792+ |
| PIK3CD chr1:9609747,9609749,9609752,9609755,9609758,9609762+ |
| PIK3CD chr1:9609909,9609912,9609920+ |
| PIK3CD chr1:9635442,9635456+ |
| SOD1 chr21:31953511+ |
| SOD1 chr21:31953619,31953622+ |
| SOD1 chr21:31853537+ |
| SOD1 chr21:31853586,31853588,31853591,31853596+ |
| SOD1 chr21:31853660,31853663+ |
| SOD1 chr21:31853837+ |
| IL8 chr4:74705162+ |

**Table 2. Univariate and multivariate analyses of umbilical cord CpG methylation in relation to child's body composition measured by dual energy X-ray absorptiometry at age 9 years**

| Childhood body composition | | | | **Variance explained** |
|---|---|---|---|---|
| ***Total fat mass** (in kg)* | **eNOS chr7:150315553+** | **RXRA chr9:136355885+** | **Sex** | |
| Univariate analysis: β, P | 0.19, *P=0.001* | 0.19, *P=0.003* | 0.33, *P=0.001* | |
| Multivariate analysis: β, P | 0.17, *P=0.002* | 0.20, *P=0.001* | 0.38, *P*=*0.001* | **44%** (n=55) |
| | | | | |
| ***Percentage fat*** | **eNOS chr7:150315553+** | **RXRA chr9:136355885+** | **Sex** | |
| Univariate analysis: β, P | 0.12, *P=0.006* | 0.13, *P=0.006* | 0.35, *P<0.001* | |
| Multivariate analysis: β, P | 0.11, *P*=*0.009* | 0.13, *P=0.005* | 0.39, *P<0.001* | **47%** (n=55) |
| | | | | |
| ***Ratio trunk*/*limb fat*** | **eNOS chr7:150315553+** | **RXRA chr9:136355885+** | **Sex** | |
| Univariate analvsis: β, P | 0.072, *P*=*0.008* | 0.069, *P*=*0.016* | 0.112, *P=0.009* | |
| Multivariate analysis: β, P | 0.079, *P=0.004* | 0.072, *P*=*0.021* | 0.107, *P*=*0.059* | 28% (n=55) |
| | | | | |
| ***Total lean mass** (kg)* | **eNOS chr7:150315553+** | **SOD1 chr21:31853837**+ | **Sex** | |
| Univariate analysis: β, P | 0.651, *P*=*0.06* | -1.037, *P*=*0.015* | -2.412, *P<0.001* | |
| Multivariate analvsis: β, P | 0.784, *P*=*0.026* | -1.136, *P=0.004* | -2.449, *P*=*0.001* | **31%** (n=60) |
| | | | | |
| ***Bone mineral content*** (kg)* | **eNOS chr7:150315553+** | **SOD1 chr21:31853837+** | **Sex** | |
| Univariate analysis: β, P | 0.053, *P=0.006* | -0.054, *P*=*0.021* | -0.081, *P*=*0. 02* | |
| Multivariate analysis: β, P | 0.057, *P*=*0.005* | -0.064, *P*=*0.005* | -0.089, *P*=*0.031* | **26%** (n=60) |
| | | | | |
| ***Bone mineral content*** (kg)* | **eNOS chr7:150315553+** | **PIK3CDchr1:9635535**/**37**+ | **Sex** | |
| Univariate analysis: β, P | 0.053, *P=0.006* | -0.087, *P=0.008* | -0.086, *P=0.02* | |
| Multivariate **analysis:** β, P | 0.082, *P=0.001* | -0.064, *P=0.029* | -0.126, *P=0.011* | **54%** (n=33) |

| | | | | |
|---|---|---|---|---|
| **Values are regression slope, β, per Z-score in CpG methylation ans P value. *Adjusted for child's age a measurement** | | | | |

### Example 1b:

### Methylation status of specific CpGs related to adiposity at ages 6 and 9

In an extension of the above noted studies reported in Example 1a, umblical cord DNA from healthy neonates was used to relate the methylation status of specific CpGs 5' from candidate genes to maternal pregnancy diet and to later childhood adiposity measured by dual energy X-ray absorptiometry (DXA). Genes selected were retinoid X receptor-alpha (RXRA), endothelial nitric oxide synthase (eNOS), superoxide dismutase-1 (SOD1), interleukin-8 (IL8) and phosphoinositide-3-kinase, catalytic, delta polypeptide (PI3KCD).

Methylation, measured by the Sequenom MassARRAY system, varied greatly at particular CpG sites. In summary, of 68 CpGs studied in relation to the age 9 DXA data, 31 had a median methylation ≥5% and a 5-95% range ≥10%. RXRA chr9:136355885+ and eNOS chr7:150315553+ methylation had independent associations with childhood fat mass (r=0.32, P=0.009, n=64 and r=0.42, P<0.001, n=66, respectively) and %fat mass (r=0.29, P=0.023, n=64 and r=0.37, P=0.002, n=66, respectively), independently of sex at age 9. Moreover, higher methylation of RXRA chr9:136355885+, but not of eNOS chr7:150315553+, was associated with lower maternal carbohydrate intake in early pregnancy, previously linked with higher neonatal adiposity in this population.

In a second independent group of 27 children selected form the Southampton Women's Survey (SWS; Inskip et al. (2006) 'Cohort profile; The Southampton Women's Survey' Int. J. Epidemiol. 35, 42-48), methylation of CpG sites in umbilical cord DNA was compared with DXA measurement of adiposity at age 6. In this group, cord eNOS chr7:150315553+ methylation showed no association with adiposity, but RXRA chr9:136355885+ methylation showed remarkably similar associations with fat mass and %fat mass (r=0.47, P=0.014 and r=0.43, P=0.027, n=27). Controlling for sex, epigenetic marks measured at birth explained >20% of the variance in adiposity in later childhood, suggesting a substantial component of metabolic disease risk has a prenatal developmental basis.

These results are of immense interest in the light of the growing amount of epidemiological evidence in humans linking factors in prenatal and early life to later adiposity and the risk of metabolic disease (Gluckman et al. (2001) 'Fetal programming and adult health', Public Health Nutrition 2(B), 611-624; Gluckman et al. (2008) Effect of in utero and early-life conditions on adult health and disease. N. Engl. J. Med. 359, 61-73). For example, as noted above, a period of severe nutritional constraint during pregnancy is associated with obesity in adulthood in the offspring (see also, Ravelli et al. (1976) 'Obesity in young men after famine exposure in utero and early infancy', New Engl. J. Med. 295, 349-353) and normal variations in maternal body composition relate to child's later adiposity (Gale et al. (2007) 'Maternal size in pregnancy and body composition in children', J. Clin Endocrinol. Metab. 92, 3904-3911). However, the relative importance of such pathways in generating individual vulnerability to obesity is unknown and understanding of the underlying mechanisms is limited. In animal models there is extensive evidence linking early life environmental changes, for example manipulation of maternal diet, to altered metabolism and body composition in adult offspring and recent studies suggest that epigenetic processes underpin these associations, providing a mechanistic explanation (Burdge et al (2007) Dietary protein restriction of pregnant rats in the F0 generation induces altered methylation of hepatic gene promoters in the adult male offspring in the F1 and F2 generations.' Br. J. Nutr. 97, 435-439; Drake et al. (2005) 'Intergenerational consequences of fetal programming by in utero exposure to glucocorticoids in rats.' Am. J..Physiol. Regul. Integr. Comp. Physiol 288, R34-38; Champgane et al. (2007) 'Transgenerational effects of social environment on variations in maternal care and behavioural response to novelty.' Behav. Neurosci.121, 1353-1363.)

In the rat, unbalanced maternal diet during pregnancy induces changes in DNA methylation and covalent histone modifications in the 5' promoter regions of specific non-parentally imprinted genes and affects the offspring's later body composition and metabolic phenotype (Lillycrop et al (2005) *ibid*; Bogdarina et al. (2007) 'Epigenetic modification of the rennin-angiotensin system in the fetal programming of hypertension', Circ. Res. 100, 520-526). In such experimental models, endocrine or nutritional interventions in early life can reverse or prevent both later phenotypic effects and the associated epigenetic changes (Gluckman et al. (2007) Proc. Natl Acad. Sci. USA 104, 12796-12800; Lillycrop et al. (2008) Br. J. Nutr. 100, 278-282).

The results summarised above now indicate that perinatal epigenetic analysis at specific selected CpGs, especially for example RXRA chr9:136355885+, may have utility in identifying individual vulnerability to later obesity. These results are further discussed below.

Supplementary Table S3 sets out the amplicons corresponding to the promoter regions of the five candidate genes which were chosen for study using DNA extracted form umbilical cord samples from 68 subjects in the PAH cohort study.

The characteristics of the PAH cohort study subjects at birth and at follow up at age 9 are shown in supplementary Table S6, together with those of the second independent group of children from the Southampton Women's Survey followed up at age 6

Analysis was restricted to CpGs with median methylation ≥5% and a 5-95% range ≥10%. Using Stata, PAH study variables were transformed where necessary to satisfy statistical assumptions of normality and Pearson correlation (rₚ) and linear regression used to examine CpG methylation in relation to mother's diet and child's adiposity, adjusting for sex. In the smaller SWS group, rankings of percentage methylation and offspring adiposity were analysed.

For the 68 CpGs and CpG groups studied, measurements were available for between 28 and 68 of the PAH subjects, and 31 CpGs met criteria (median methylation ≥5% and 5-95% range ≥10%) for further analysis (Supplementary Table S5) Particularly marked inter-individual variation in the degree of methylation was seen at some sites (for example, RXRA chr9:136355885+ had a median of 59% with 5^{th}-95^{th} centiles 4-99%; eNOS chr7:150315553+ had a median of 93%, with 5^{th}-95^{th} centiles 64-100%). Correlations between methylation of different CpGs were generally low (for example rₚ=0.03, P=0.81, n=55 between the above two CpGs).

Of the 31 CpGs for which variable methylation above the *a priori* threshold was found in umbilical cord DNA, seven had significant associations with DXA measures of the child's adiposity at age 9 years (Supplementary Material, Table S7). Table 3 below shows the results of uni- and multivariate analyses of CpG sites with significant independent associations with the child's adiposity. RXRA chr9:136355885+ and eNOS chr7:150315553+ methylation had independent positive associations with childhood fat mass (r=0.32, P=0.009, n=64 and r=0.42, P<0.001, n=66, respectively), %fat mass (r=0.28, P=0.023, n=64 and r=0.37, P=0.002, n=66, respectively) and ratio of trunk/limb fat (r=0.26, P=0.039, n=64 and r=0.33, P=0.007, n=66, respectively), independently of sex. Methylation of eNOS chr7:150315553+ and RXRA chr9:136355885+ had similar positive associations with child's body mass index (rₚ=0.39, P=0.001 and rₚ=0.26, P=0.037, respectively). Independently of sex, eNOS chr7:150315553+ and RXRA chr9:136355885+ methylation, SOD1 chr21:31853660/63+ methylation was also inversely related to child's trunk/limb fat ratio (P=0.037). The associations reflect clinically important shifts in adiposity such that the mean sex-adjusted fat mass rose from 4.8 kg (17.3% body fat) in the lowest quarter of RXRA chr9:136355885+ methylation to 6.6 kg (21.3% body fat) in the highest quarter of the distribution. Taking account of RXRA chr9:136355885+ methylation and sex explained 26% of the variance in fat mass in PAH children and simultaneous analyses showed that the association between RXRA chr9:136355885+ methylation and child's fat mass was independent of birth weight and mother's body mass index.

**Table 3. Univariate and multivariate analyses of umbilical cord CpG methylation in relation to child's adiposity measured by dual energy X-ray absorptiometry at age 9 years (see also in Table 2).**

| | | | | **Variance explained** |
|---|---|---|---|---|
| ***Total fat mass** (In kg)* | **eNOS chr7:150315553+** | **RXRA chr9:136355885+** | **Sex** | |
| Univariate analysis | 0.19, *P=0.001* | 0.19, *P=0.003* | 0.33, *P=0.001* | |
| Multivariate analysis | 0.17, *P=0.002* | 0.20, *P=0.001* | 0.38, *P=0.009* | **44%** (n=55) |
| | | | | |
| ***Percentage fat** (In* %) | **eNOS chr7:150315553+** | **RXRA chr9:136355885+** | **Sex** | |
| Univariate analysis | 0.12, *P=0.006* | 0.13, *P=0.006* | 0.35, *P<0. 001* | |
| Multivariate analysis | 0.11, *P=0*.*009* | 0.13, *P=0.005* | 0.39, *P<0.001* | **47% (n=55)** |
| | | | | |
| ***Ratio trunk***/***limb fat*** | **eNOS chr7:150315553+** | **RXRA chr9:136355885+** | **Sex** | |
| Univariate analysis | 0.072, *P=0.008* | 0.069, *P=0.016* | 0.112, *P=0. 009* | |
| Multivariate analysis | 0.079, *P=0.004* | 0.072, *P=0.021* | 0.107, *P=0.059* | **28% (**n=55) |

| | | | | |
|---|---|---|---|---|
| Values are regression slope,β, per Z-score change in CpG methylation and P value | | | | |

In this population, lower maternal carbohydrate intake in early pregnancy had previously been linked with higher neonatal adiposity (Godfrey et al. (1997) 'Maternal birthweight and diet pregnancy in relation to the infant's thinness at birth', Br. J. Obset.

Gynaecol. 104, 663-667). Relating umbilical cord CpG methylation to maternal carbohydrate intake in early pregnancy, it was found that higher methylation of RXRA chr9:136355885+, but not of eNOS chr7:150315553+, was associated with a lower maternal carbohydrate intake (Fig. 7a). Relating umbilical cord CpG methylation to the infant's size at birth, only three CpGs showed a significant correlation with birth weight adjusted for sex and gestational age (PIK3CD chr1:9609980+ (rₚ=-0.32, P=0.013, n=58), PIK3CD chr1:9635676/79+ (rₚ=-0.36, p=0.028, n=37) and SOD1 chr 21:31853827+ (rₚ=-0.27, p=0.029, n=65)).

As follow-up, it was sought to replicate the associations between umbilical cord eNOS chr7:150315553+ and RXRA chr9:136355885+ CpG methylation and child's adiposity in a second independent group of children selected from the SWS cohort on the basis of having both umbilical cord DNA available and DXA measurement of adiposity age 6 years. In these 27 subjects, eNOS chr7:150315553+ showed no association with adiposity, but RXRA chr9:136355885+ showed remarkably similar associations to those in PAH children (Fig. 7b, c, d).

Finally, the presence of a RXRA chr9:136355885+ single nucleotide polymorphism (SNP) confounding the analysis was excluded by sequencing in SWS subjects. SNP analysis was by pyrosequencing using the PyroMark Q96MD. Briefly, this method is based on indirect luminometric quantification of the pyrophosphate that is released as a result of nucleotide incorporation into the amplicon of interest (Algerhorn et al. Gen Res. 10, 1249-1258)

### Discussion

This study thus provides novel evidence regarding the importance of the developmental contribution to later adiposity. More particularly, greater methylation of RXRA chr9:136355885+ measured at birth was strongly correlated with greater adiposity in later childhood in two independent cohorts.

It is noteworthy that the genes for which we report effects are not parentally imprinted. Hence, although in vitro fertilisation increases risk of imprinting disorders and effects on imprinted genes have been reported in offspring of mothers exposed to famine during early pregnancy, the present study adds evidence to implicate the human prenatal environment with epigenetic changes in non-imprinted genes and connects epigenetic state at birth with clinically relevant later phenotypic variation.

Genome-wide association studies suggest that fixed genetic variation makes a relatively small contribution to obesity, heart disease and diabetes; the findings of the inventors raise the possibility that the developmental component may be equally or more important. As noted above, the presence of a SNP at RXRA chr9:136355885+ was excluded by sequencing, but without genome-wide analysis it is not possible to exclude a genetic effect of distant SNPs which could influence both DNA methylation of a particular sequence and child's phenotype. However, even if this were the case, our data clearly indicate the prognostic value of epigenetic measures at birth.

DNA methylation is the most stable of epigenetic states and the profile appearing during development reflects the influence of environmental factors acting on the genotype. Such changes can be tissue specific and in this respect the umbilical cord may be advantageous because it contains a high proportion of fetal vascular tissue and mesenchymal cells which may be relevant to later adiposity.

Variation in the degree of methylation of non-imprinted genes and in later cardiovascular and metabolic physiology can be induced experimentally by manipulation of the developmental environment, for example by altering maternal nutrition or administering glucocorticoids during pregnancy, often independently of effects on birth weight. Whilst associations between some epigenetic markers and birth weight were found, these were weaker than the associations with later phenotype, and previous reports on the same cohort showed only modest associations between birth weight and later body composition (Gale et al. (2007) *ibis*). Because birth weight in humans is influenced by multiple factors including the mother's own birth weight and height and by gestational length, epigenetic changes may provide a more sensitive index than birth weight of environmentally-induced effects on fetal development.

The data indicate possible mechanistic pathways, suggesting avenues for future study. The observation that adjacent or nearby CpGs within the same promoter showed differences in the strength of association with child's adiposity suggests highly specific changes in the transcriptional regulation of these genes induced by the developmental environment, rather than generalised changes in promoter methylation. Both CpG hyper- and hypo-methylation at different sites predicted body fat distribution, again indicating complexity in transcriptional control.

The specificity of the association between methylation of an individual CpG and both maternal diet and child's phenotype endorses the concept of a fine control of development by environmental factors via epigenetic processes. The data for RXRA chr9:136355885+ presented herein indicates one potential mechanistic pathway involved, because induction of transcription by RXRA is dependent on ligands including peroxisome proliferator activated receptors, involved in insulin sensitivity, adipogenesis and fat metabolism (Alvarez et al. (2000) Biochem. J. 345, 91-97; Sugden & Holness (2008) Biochem. Soc. Trans. 36, 891-900)). Moreover, RXRA chr9:136355885+ is located in a region proposed to contain positive regulatory elements of transcription (Li et al. (2006) Gene 372, 118-127). Fig. 8 shows the proximity of RXRA chr9:136355885+ to proposed binding sites for RXR, MAF, NFκB and AP1.

The implications of the strong association between RXRA chr9:136355885+ methylation and later adiposity, replicated in an independent cohort, are profound. First, the effect is much larger than that of factors such as birth weight or maternal body composition, suggesting that perinatal epigenetic markers may be very useful predictors of later obesity. Secondly, the association between CpG methylation and child's adiposity operates within the normal ranges of maternal nutritional state and of birth size. The association between RXRA chr9:136355885+ methylation and mother's carbohydrate intake raises the possibility that conditions in early pregnancy could affect child's adiposity through this pathway. This provides additional support for the argument that all women of reproductive age should have appropriate nutritional, education and lifestyle support to improve the health of the next generation. Thirdly, the data provide strong evidence supporting a role for developmental plasticity in determining individual risk of metabolic disease.

### Comparative Example 1

### Childhood lean mass, bone mineral content and height at age 9 years

For 20 of the 37 CpGs/CpG groups, evidence was found that the degree of methylation was associated with the child's total lean body mass and/or proportionate lean mass and/or bone mineral content and/or height (Table 4). Figure 2a shows a positive correlation of umbilical cord eNOS chr7:150315553+ methylation and an inverse correlation of SOD1 chr21:31853837+ methylation with the child's lean mass, adjusted for sex, and Figure 2b that eNOS chr7:150315553+ methylation had a positive correlation and SOD1 chr21:31853837+ methylation had an inverse correlation with bone mass, adjusted for sex. Similarly, the child's height, adjusted for sex, was positively associated with eNOS chr7:150315553+ methylation and inversely with SOD1 chr21:31853837+ methylation (rp=0.23, P=0.062 and rₚ=-0.29, P=0.019, respectively). There were important differences in lean and bone mass across the distributions of umbilical cord CpG methylation; for example, mean sex-adjusted lean mass and bone mineral content fell from 23.3 kg and 1.23 kg, respectively, in the lowest quarter of SOD1 chr21:31853837+ to 20.9 kg and 1.09 kg in the highest quarter of the distribution. Table 2 shows univariate and multivariate analyses of umbilical cord CpGs whose methylation levels had significant independent associations with the child's lean and bone mass. Taking account of the child's sex, methylation status of eNOS chr7:150315553+ and SOD1 chr21:31853837+ explained 31% of the variance in the child's lean mass and 26% of that in bone mineral content at age 9 years. Measurements of both eNOS chr7:150315553+ and phosphoinositide-3-kinase, catalytic, delta polypeptide (PIK3CD) chr1:9635535/37+ methylation were only available for 30 children, but taking account of the child's sex they explained 54% of the variance in bone mineral content (Table 2). PIK3CD chr1:9635535/37+ methylation was also strongly associated with the child's height (rₚ=-0.44, P=0.006, n=37).

**Table 4: CpGs/CpG groups whose methylation was associated with the child's total lean body mass and/or proportionate lean mass and/or bone mineral content and/or height identified by genomic coordinates (UCSC, human genome March 2006 assembly)**

| |
|---|
| eNOS chr7:150315553+ |
| eNOS chr7:150306792+ |
| LPL chr8:19840701 |
| PIK3CD chr1:9609747,9609749,9609752,9609755,9609758,9609762+ |
| PIK3CD chr1:9609870+ |
| PIK3CD chr1:9609909,9609912,9609920+ |
| PIK3CD chr1:9609980+ |
| PIK3CD chr1:9635515,9635587+ |
| PIK3CD chr1:9635535,9635537+ |
| SOD1 chr21:31953511+ |
| SOD1 chr21:31853537+ |
| SOD1 chr21:31853660,31853663+ |
| SOD1 chr21:31853837+ |
| RXRA chr9:136355556,136355560+ |
| RXRA chr9:136355593,136355600+ |
| RXRA chr9:136355688+ |
| RXRA chr9:136355836+ |
| RXRA chr9:136355885+ |
| RXRA chr9:136357196+ |
| IL8 chr4:74705162+ |

### Comparative Example 2

### Childhood cardiovascular structure and function at age 9 years

Left ventricular mass, aortic root diameter, coronary artery diameter, carotid artery intima-media thickness, systolic and diastolic blood pressure, pulse rate and pulse wave velocity were used as objective indicators of cardiovascular structure and function and correlations found with the methylation state of specific CpGs. For 30 of the 37 CpGs/CpG groups studied, evidence was found that the degree of methylation was associated with the cardiovascular parameters stated above (Table 5). Figure 3a shows the strong inverse association between PIK3CD chr1:9609870+ methylation and child's carotid artery intima-media thickness and Figure 3b the positive association between eNOS chr7:150306798+ methylation and aorto-femoral pulse wave velocity. Table 5 shows the results of univariate and multivariate analyses in relation to child's cardiovascular structure and function. Taking account of the child's sex, methylation level of SOD1 chr21:31853837+ explained 23% of the variance in left ventricular mass; methylation levels of SOD1 chr21:31853837+ and PIK3CD chr1:9609870+ explained 36% of the variance in the child's carotid artery intima-media thickness; SOD1 chr21:31853837+ was also related to aortic root diameter (rₚ=-0.33, P=0.008, n=65), and total coronary artery diameter was related to PIK3CD chr1:9635515/87+, SOD1 chr21:31953619/22+ and RXRA chr9:136355836+ methylation (rₚ=-0.42, P=0.028, n=28; rₚ=0.46, P=0.019, n=26; and rₚ=0.41, P=0.004, n=48, respectively). Measurement of SOD1 chr21:31853537+ methylation was only available for 30 children but explained 28% of the variance in aorto-foot pulse wave velocity. Methylation levels of eNOS chr7:150306798+ and RXRA chr9:136355688+ explained 17% of the variance in the child's aorto-femoral pulse wave velocity at age 9 years. Taking account of the child's sex and age, the CpG groups PIK3CD chr1:9609747/49/52/55/58/62+ and PIK3CD chr1:9609909/12/20+ explained 23% of the variance in systolic blood pressure (Table 6).

**Table 5: CpGs/CpG groups whose methylation was associated with the child's cardiovascular structure and function identified by genomic coordinates (UCSC, human genome March 2006 assembly)**

| |
|---|
| eNOS chr7:150306792+ |
| eNOS chr7:150306798+ |
| eNOS chr7:150315553+ |
| eNOS chr7:150315604+ |
| PPARg2 chr3:12367795+ |
| PPARg2 chr3:12367792+ |
| PPARg2 chr3:12367759+ |
| PIK3CD chr1:9609747,9609749,9609752,9609755,9609758,9609762+ |
| PIK3CD chr1:9609870+ |
| PIK3CD chr1:9609909,9609912,9609920+ |
| PIK3CD chr1:9609980+ |
| PIK3CD chr1:9635442,9635456+ |
| PIK3CD chr1:9635515,9635587+ |
| PIK3CD chr1:9635535,9635537+ |
| PIK3CD chr1:9635676,9635679+ |
| SOD1 chr21:31951522+ |
| SOD1 chr21:31953394+ |
| SOD1 chr21:31953511+ |
| SOD1 chr21:31953619,31953622+ |
| SOD1 chr21:31853537+ |
| SOD1 chr21:31853586,31853588,31853591,31853596+ |
| SOD1 chr21:31853660,31853663+ |
| SOD1 chr21:31853837+ |
| RXRA chr9:136355569+ |
| RXRA chr9:136355688+ |
| RXRA chr9:136355836+ |
| RXRA chr9:136355885+ |
| RXRA chr9:136357082,136357085,136357087+ |
| IL8 chr4:74705041,74705043,74705045,74705049+ |
| IL8 chr4:74705094+ |

**Table 6. Univariate and multivariate analyses of umbilical cord CpG methylation in relation to child's cardiovascular structure and function at age 9 years**

| ***Cardiovascular structure*** | | | | | **Variance explained** |
|---|---|---|---|---|---|
| ***Left ventricular mass (g)*** | **SOD1 chr21:31853837**+ | | **Sex** | | |
| Univariate analysis: β, P | -7.7, *P=0.002* | | -13.5, *P<0.001* | | |
| Multivariate analysis: β, P | -7.2, *P=0.003* | | -11.4, *P=0.009* | | **23%** (n=65) |
| | | | | | |
| ***Carotid intima-media** thickness **(mm)*** | **SOD1 chr21:31853837**+ | **PIK3CD chr1:9609870**+ | **Sex** | | |
| Univariate analysis: β, P | -0.017, *P=0.034* | -0.033, *P<0.001* | -0.005, *P*=*0.68* | | |
| Multivariate analysis: β, P | -0.013, *P=0.079* | -0.033, *P<0.001* | | | **36%** (n=49) |
| ***Cardiovascular function*** | | | | | |
| ***Aorto-foot pulse wave velocity** (m*/*sec)* | **SOD1 chr21:31853537**+ | | **Sex** | | |
| Univariate analysis: β, P | -0.157, *P=0.004* | | -0.108, *P=0.92* | | |
| Multivariate analysis: β, P | -0.166, *P=0.003* | | -0.088, *P=0.35* | | **28%** (n=31) |
| | | | | | |
| ***Aorto-femoral pulse wave velocity (m*/*sec)*** | **eNOS chr7:150306798+** | **RXRA chr9:136355688+** | **Sex** | | |
| Univariate analysis: β, P | 0.071, *P=0.051* | -0.073, *P=0.046* | 0.01, *P=0.80* | | |
| Multivariate analysis: β, P | 0.079, *P=0.05* | -0.104, *P=0.010* | | | **17%** (n=52) |
| | | | | | |
| ***Systolic blood pressure** (mm Hg)* | PIK3CD **chr1:9609747**/**49**/**52**/**55**/**58**/**62**+ | **PIK3CD chr1:9609909/12/20+** | **Sex** | **Age** | |
| Univariate analysis: β, P | 2.53, *P=0.022* | 2.43, *P=0.03* | 2.37, *P=0.14* | 6.42, *P=0.063* | |
| Multivariate analysis: β, P | 2.36, *P=0.026* | 2.34, *P=0.029* | 1.50, *P=0.45* | 9.94, *P=0.049* | **23%** (n=58) |

| | | | | | |
|---|---|---|---|---|---|
| Values are regression slope, β, per Z-score change in CpG methylation and P value | | | | | |

### Comparative Example 3

### Childhood IQ at age 9 years

For 16 of the 37 CpGs/CpG groups, we found evidence that the degree of methylation was associated with child's IQ (Table 7). The associations with the child's verbal IQ were particularly strong for SOD1 chr21:31953511+ (Spearman correlation coefficient r=-0.43, P=0.015, n=32) and IL8 chr4:74705162+ (r=-0.36, P=0.017, n=45). The associations with the child's performance IQ were particularly strong for eNOS chr7:150306792+ (Spearman correlation coefficient rₛ=-0.29, P=0.015, n=72), PIK3CD chr1:9609980+ (rₛ=0.27, P=0.04, n=56) and SOD1 chr21:31953394+ (rₛ=0.44, P=0.015 n=30). The associations with the child's full scale IQ were particularly strong for eNOS chr7:150306792+ (Spearman correlation coefficient rₛ=-0.27, P=0.022, n=72), PIK3CD chr1:9635535/37+ (rₛ=-0.34, P=0.037, n=37), SOD1 chr21:31953394+ (rₛ=0.39, P=0.033 n=30, Figure 4), SOD1 chr21:31953511+ (rₛ=-0.36, P=0.042, n=32) and RXRAchr9:136355885+ (rₛ=0.25, P=0.049, n=63).

**Table 7: CpGs/CpG groups whose methylation was associated with the child's IQ identified by genomic coordinates (UCSC, human genome March 2006 assembly)**

| |
|---|
| eNOS chr7:150306792+ |
| eNOS chr7:150315604+ |
| PPARg2 chr3:12367795+ |
| PIK3CD chr1:9609870+ |
| PIK3CD chr1:9609980+ |
| PIK3CD chr1:9635515,9635587+ |
| PIK3CD chr1:9635535,9635537+ |
| PIK3CD chr1:9635676,9635679+ |
| SOD1 chr21:31953394+ |
| SOD1 chr21:31953511+ |
| SOD1 chr21:31853837+ |
| RXRA chr9:136355569+ |
| RXRA chr9:136355836+ |
| RXRA chr9:136355885+ |
| IL8 chr4:74705094+ |
| IL8 chr4:74705162+ |

### Comparative Example 4

### Childhood neuro-behavioural status at age 9 years

Results also indicate that degree of specific CpG/ CpG group methylation can be used as a useful marker for neuro-behavioural disorders including, but not limited to hyperactivity, emotional problems, conduct problems, peer problems and/or total difficulties. For 23 of the 37 CpGs/CpG groups studied, evidence was found that the degree of methylation was associated with child's score on hyperactivity, emotional problems, conduct problems, peer problems and/or total difficulties scales (Table 8). The associations with hyperactivity score were particularly strong for the PPARg2 chr3:12367759+ (r=-0.41, P<0.001, n=67; Figure 5a) and RXRA chr9:136355885+ (r=-0.38, P=0.002, n=63; Figure 5b), and there were similar associations with the total difficulties score: PPARg2 chr3:12367759+ (r=-0.36, P=0.003, n=66) and RXRA chr9:136355885+ (r=-0.44, P<0.001, n=62). The associations with the conduct problem scores were particularly strong for PIK3CD chr1:9609980+ (r=-0.38, P=0.003, n=58), RXRA chr9:136355688+ (r=-0.27, P=0.030, n=64) and RXRA chr9:136357196+ (r=0. 28, P=0.035, n=59). The associations with peer problems were particularly strong for RXRA chr9:136355836+ (r=-0.28, P=0.026, n=63), RXRA chr9:136355885+ (r=-0.29, P=0.021, n=63) and RXRA chr9:136357082/85/87+ (r=-0. 26, P=0.038, n=62). Prosocial behaviour was particularly associated with IL8 chr4:74705094+ methylation (r=-0.36, P=0.014, n=46).

**Table 8: CpGs/CpG groups whose methylation was associated with the child's neurobehavioural status identified by genomic coordinates (UCSC, human genome March 2006 assembly)**

| |
|---|
| eNOS chr7:150306792+ |
| eNOS chr7:150315604+ |
| PPARg2 chr3:12367795+ |
| PPARg2 chr3:12367759+ |
| PIK3CD chr1:9609870+ |
| PIK3CD chr1:9609909,9609912,9609920+ |
| PIK3CD chr1:9609980+ |
| PIK3CD chr1:9635515,9635587+ |
| PIK3CD chr1:9635535,9635537+ |
| PIK3CD chr1:9635676,9635679+ |
| SOD1 chr21:31953263,31953483+ |
| SOD1 chr21:31853660,31853663+ |
| SOD1 chr21:31853837+ |
| RXRA chr9:136355556,136355560+ |
| RXRA chr9:136355569+ |
| RXRA chr9:136355593,136355600+ |
| RXRA chr9:136355688+ |
| RXRA chr9:136355836+ |
| RXRA chr9:136355885+ |
| RXRA chr9:136357082,136357085,136357087+ |
| RXRA chr9:136357196+ |
| IL8 chr4:74705094+ |
| IL8 chr4:74705162+ |

### Comparative Example 5

### Atopic eczema and risk of allergic disease

For 5 of the 13 CpGs/CpG groups with methylation measurements in four or more atopic eczema cases, evidence was found that the degree of methylation (determined by pyrosequencing) was associated with atopic eczema in infancy as determined with reference to the UK working party criteria for diagnosis of atopic eczema (Table 9). The associations with atopic eczema were particularly strong for eNOS chr7:150306792+ and eNOS chr7:150306798+ (Figure 6). Methylation status of specifc CpGs/CpG groups is therefore of interest in predicting propensity for atopic eczema and other allergic disorders. Identification of such association may enable intervention to reduce the risk of development or seriousness of such disorders.

**Table 9: CpGs/CpG groups whose methylation was associated with atopic eczema in infants at age 9 months (n=74) identified by genomic coordinates (UCSC, human genome March 2006 assembly)**

| |
|---|
| eNOS chr7:150306792+ |
| eNOS chr7:150306798+ |
| PIK3CD chr1:9609870+ |
| PIK3CD chr1:9635535,9635537+ |
| RXRA chr9:136355885+ |

### Summary of findings - specificity and CpG "fingerprinting"

The associations with outcome were linked to specific CpGs 5' to the start site of candidate genes. There are examples in which adjacent or nearby CpGs showed differences not only in the strength but also in the direction of association with child's phenotype: the CpG closest to eNOS chr7:150315553+ (eNOS chr7:150315604+) showed substantial variation but no associations with the child's fat mass (rₚ=0.01, P=0.94), percentage fat mass (rₚ=0.03, P=0.83) or trunk/limb fat ratio (rₚ=0.09, P=0.50); SOD1 chr21:31953511+ was inversely associated with trunk/limb fat ratio, whereas SOD1 chr21:31953619/22+ was positively associated with this ratio (supplementary table S7); SOD1 chr21:31953511+ was inversely associated with the child's full scale IQ (rₛ=-0.36, P=0.042), whereas the neighbouring CpG SOD1 chr21:31953394+ was positively associated with full scale IQ (rₛ=0.39, P=0.033). This suggests highly specific changes in the transcriptional regulation of these genes induced by the developmental environment rather than generalised changes in promoter methylation. CpG hyper- and hypomethylation both related significantly to outcomes, again indicating complexity in the transcriptional control. Beyond these simple associations, multivariate analysis indicated that the associations were independent of gender and birth weight, and established the degree to which outcome could be attributed to the combined effects of levels of DNA methylation at specific CpGs/CpG groups across the same and different genes (Tables 2 and 6). The reported high correlations suggest important developmental contributions to disease risk.

Of the CpGs shown in Tables 2 and 6, RXRA chr9:136355885+, RXRA chr9:136355688+, eNOS chr7:150315553+, eNOS chr7:150306798+ and PIK3CD chr1:9635535/37+ are either within the proximal promoter or close to it. While the other CpGs in intergenic regions may exert effects through regulation of other genes, the data nonetheless indicate possible mechanistic pathways, suggesting avenues for future studies. Each of the genes examined is biologically plausible in relation to the outcomes measured. Superoxide dismutase 1 (SOD1) contributes to defence against oxidative stress, implicated in the aetiology of vascular pathology (Cai and Harrison (2000) Cir. Res. 87, 840-844). eNOS regulates vasodilation and expression is reduced in animal models of developmental induction of hypertension (Torrens et al (2006) Hypertension 47, 982-987). Phosphoinositide--3-kinase mediates multiple cell signalling systems, including insulin action, and the delta polypeptide is a catalytic subunit (Marone et al. (2008) Biochimica et Biophysica Acta 1784, 159-185). As previously noted above, RXRA is a ligand-dependent transcription factor, with ligands including peroxisome proliferator activated receptors α and y, involved in insulin sensitivity, adipogenesis and fat metabolism (Alvarez et al (2000) Biochem. J. 345, 91-97; Sugden et al. (2008) Biochem. Soc. Trans. 36, 891-900.) Interleukin-8 (IL-8) is a chemokine which has angiogenic activity and may play a role in metabolic disease and vascular dysfunction (Waugh and Wilson (2008) Clin. Cancer Res. 14, 6735-41; Kim et al. (2006) Int. J. Obes. (Lond.) 30, 1347-55). It is noteworthy that these genes are not parentally imprinted genes. Hence, although *in vitro* fertilisation increases risk of imprinting disorders and prenatal famine can cause epigenetic effects on an imprinted gene, the studies reported above are the first to implicate the prenatal environment with epigenetic changes in non-imprinted genes.

As indicated above, strong associations have been identified between methylation measures at birth and various phenotypic precursors of metabolic and cardiovascular disease in later childhood. Taking the level of significance as 0.005 to take account of multiple comparisons, the following associations are of particular note: methylation status of RXRA chr9:136355885+ with fat mass, lean mass and bone mineral content; methylation status of eNOS chr7:150315553+ with fat mass, trunk/limb fat ratio and bone mineral content; methylation status of SOD1 chr21:31853837+ with lean and left ventricular mass; methylation status of SOD1 chr21:31853537+ with pulse wave velocity; and methylation status of PIK3CD chr1:9609870+ with carotid intima-media thickness.

The implication of studies reported above is that up to 50% of the variation in body composition, cardiovascular structure and function in pre-pubertal children can be explained by epigenetic measurements at birth, suggesting that the developmental environment exerts a strong influence on predisposition to later chronic disease. Epigenetic methylation markers measured at birth are thus envisaged as providing highly valuable early prognostic indicators of susceptibility to later disease, which could be used to devise and monitor interventions.

**Supplementary Table S1: Characteristics of the 87 children at age 9 years**

| | **Median (IQR)** | **No. subjects** |
|---|---|---|
| Age, years | 8.67 (8.52 - 8.75) | 87 |
| ***Body composition*** | | |
| Body mass index, kg/m² | 16.3 (15.7 - 18.1) | 87 |
| DXA total fat mass, kg | 5.43 (3.97 - 8.41) | 87 |
| DXA %body fat | 18.4 (14.4 - 27.0) | 87 |
| DXA ratio of trunk/limb fat | 0.63 (0.56 - 0.78) | 87 |
| DXA total lean mass, kg | 21.8 (19.4 - 23.9) | 87 |
| DXA %body lean | 77.6 (69.4 - 81.4) | 87 |
| DXA bone mineral content, kg | 1.14 (1.03 -1.25) | 87 |
| Height, m | 1.32 (1.26 - 1.35) | 87 |
| ***Cardiovascular structure***/***function*** | | |
| Left ventricular mass, g | 82 (72 - 93) | 87 |
| Aortic root diameter, cm | 2.18 (2.07 - 2.33) | 87 |
| Total coronary artery diameter, mm | 4.2 (4.1 - 4.4) | 64 |
| Systolic blood pressure, mm Hg | 102.0 (97.3 - 106.3) | 86 |
| Diastolic blood pressure, mm Hg | 59.8 (56.0 - 63.7) | 86 |
| Carotid artery intima-media thickness, mm | 0.35 (0.31 - 0.39) | 87 |
| Aortic/femoral pulse wave velocity, m/sec | 2.74 (2.54 - 2.90) | 86 |
| Aortic/foot pulse wave velocity, m/sec | 4.67 (4.50 - 4.91) | 85 |

| | | |
|---|---|---|
| IQR = inter-quartile range; DXA = dual energy X-ray absorptiometry | | |

**Supplementary Table S2: Pyrosequencing primers**

| **Amplicon Name** | **Genomic co-ordinates (UCSC, human genome March 2006 assembly)** | **Primer Pair 5'-3' (F, R)** | **Sequencing primers 5'-3'** | **PCR anneal temp (°C)** | **Amplicon length (bp)** |
|---|---|---|---|---|---|
| LPL | Chr8:19840640-19840837 | GAGTGAGGGAGGATTGTAA GTGAT (SEQ ID NO: 1) | ATTGTAAGTGATAAATA GGA (SEQ ID NO: 2) | 50 | 198 |
| | + Strand | AACTTAAAAAATTCCACTCT ACCC (SEQ ID NO: 3) | | | |
| | | | | | |
| PPARg2 | Chr3:12367694-12367978 | ATAAAAAATGTAAGTGGATA TTGA (SEQ ID NO: 4) | AAATATTACCACACTATC TC (SEQ ID NO: 5) | 50 | 285 |
| | + Strand | CCCATAAATCAAAACATCAA TTTC (SEQ ID NO: 6) | | | |
| | | | | | |
| eNOS | Chr7:150306546-150306826 | GGAATTTTGGTAGAGGGAA ATTAG (SEQ ID NO: 7) | TTCAAATCAAATTTCTTA AT (SEQ ID NO: 8) | 56 | 280 |
| | + Strand | CCTATTTCCAAAATCTCCCT TAAC (SEQ ID NO: 9) | TATATACCAAACCAATAC TC (SEQ ID NO: 10) | | |
| | | | CAAAATCTCCCTTAACT CC (SEQ ID NO: 11) | | |
| | | | | | |
| SOD1 | Chr21:31851699 to 31852086 | GGGATTATTTAGTTTGTGAG TGAT (SEQ ID NO: 12) | CTCCTAATCTTACCCCC (SEQ ID NO: 13) | 50 | 388 |
| | + Strand | TATCCCAACCTAAAATCCAA AC (SEQ ID NO: 14) | GGGGGTAAGATTAGGA G (SEQ ID NO: 15) | | |

**Supplementary Table S3: Sequenom amplicons**

| **Amplicon Name** | **SEQ ID NO** | **Genomic co-ordinates (UCSC, human genome March 2006 assembly)** | **Primer Pair 5'-3' (F, R)** | **PCR anneal temp (°C)** | **Amplicon length (bp)** |
|---|---|---|---|---|---|
| PIK3CD CpG30 AMP01 | 16 17 | Chr1:9609665-9610090 + Strand | aggaagagagTTTATTTTTTATTTTTGGAGGAAG cagtaatacgactcactatagggagaaggctCAAAAATTCAAACCCCAACCTATTA | 56 | 426 |
| PIK3CD CpG129 AMP04 | 18 19 | Chr1:9635246-9635778 + Strand | aggaagagagTAGTTTGAATGAGGTTTTTAAAAA cagtaatacgactcactatagggagaaggctAAAAAATAATTACCCCTTCCCACTC | 56 | 533 |
| SOD1 S/H AMP1 | 20 21 | Chr21:31951294-31951584 + Strand | aggaagagagTGTGGAATTTTTGGATTTTTTTTAGTT cagtaatacgactcactatagggagaaggctTTACAAATATAAACCACCACACCC | 55 | 291 |
| SOD1 CpG91 AMP01 | 22 23 | Chr21:31953207-31953661 + Strand | aggaagagagTTGTGGTTTTTAGGTTAGATAAAAA cagtaatacgactcactatagggagaaggctATAAAAAAACAACCTTCTTTTCAC | 56 | 455 |
| SOD1 CpG91 AMP04 | 24 25 | Chr21:31954322-31954698 + Strand | aggaagagagGTGTTGTTTTTGTGGTTTTTGG cagtaatacgactcactatagggagaaggctTCTACCCTTACAACCCAATCATAAC | 55 | 377 |
| SOD1 CpG249 AMP06 | 26 27 | Chr21:31853279-31853715 + Strand | aggaagagagGGAGTTGTTAGGGTTTTTT cagtaatacgactcactatagggagaaggctCCATTAACCACCTCCACCTATAC | 58 | 437 |
| SOD1 CpG249 AMP07 | 28 29 | Chr21:31853616-31853913 + Strand | aggaagagagTTTAATAAAGTAGTTTTGGGAGGGT cagtaatacgactcactatagggagaaggctCTATCTCCCCCTAAAACCAAACAC | 58 | 298 |
| RXRA S/H AMP1 | 30 31 | Chr9:136355518-136355938 + Strand | aggaagagagGGTTGGATGTGAGTTTAGATTTGTAG cagtaatacgactcactatagggagaaggctAAAAAAATTCCATCAAACAATAACC | 55 | 421 |
| RXRA S/H AMP4 | 32 33 | Chr9:136357060-136357336 + Strand | aggaagagagTGTTTAGGAAGGTTGGGTTTGG cagtaatacgactcactatagggagaaggctCACTCCTAAAACCCCTCTTCAA | 62 | 277 |
| eNOS S/H AMP2 | 34 35 | Chr7:150315427-150315678 + Strand | aggaagagagGGGTTTAGATGAGGAGGTTGGTTA cagtaatacgactcactatagggagaaggctACAAACCCCTACCACTACCAAAATA | 62 | 252 |
| IL-8 CpG20 AMP01 | 36 37 | Chr4:74704821-74705226 + Strand | aggaagagagTTTTAGGGAGTGGATTAGAAAGTTTTT cagtaatacgactcactatagggagaaggctCACTCCCAACATACACTAAAACACAA | 56 | 406 |

**Supplementary Table S4: Distributions of CpGs measured by pyrosequencing (highlighted rows 75^{th} centile ≥5% and 5^{th}-95^{th} centiles ≥10%)**

| | **5th centile** | **25th centile** | **Median** | **75th centile** | **95th centile** | **No. subjects** |
|---|---|---|---|---|---|---|
| eNOS chr7:150306598+ | **3** | **4** | **5** | **6** | **7** | **71** |
| eNOS chr7:150306609+ | 3 | 4 | 4 | 5 | 6 | 71 |
| eNOS chr7:150306613+ | 4 | 5 | 6 | 7 | 8 | 71 |
| eNOS chr7:150306652+ | 4 | 5 | 6 | 7 | 10 | 70 |
| eNOS chr7:150306680+ | 3 | 3 | 4 | 5 | 6 | 75 |
| eNOS chr7:150306683+ | 6 | 8 | 10 | 11 | 13 | 76 |
| eNOS chr7:150306711+ | 2 | 3 | 4 | 5 | 8 | 76 |
| eNOS chr7:150306714+ | 3 | 4 | 6 | 7 | 10 | 76 |
| eNOS chr7:150306740+ | 5 | 7 | 8 | 9 | 13 | 74 |
| eNOS chr7:150306742+ | 8 | 10 | 11 | 13 | 17 | 74 |
| eNOS chr7:150306766+ | 4 | 6 | 7 | 8 | 11 | 74 |
| eNOS chr7:150306774+ | 5 | 6 | 7 | 8 | 11 | 74 |
| eNOS chr7:150306786+ | 2 | 3 | 3 | 4 | 5 | 74 |
| **eNOS chr7:150306792+** | **7** | **8** | **9** | **12** | **17** | 74 |
| **eNOS chr7:150306798+** | **4** | **7** | **9** | **10** | **15** | 74 |
| SOD1 chr21:31851951+ | 8 | 9 | 11 | 12 | 14 | 52 |
| SOD1 chr21:31851953+ | 6 | 7 | 8 | 10 | 13 | 53 |
| SOD1 chr21:31851955+ | 8 | 11 | 13 | 14 | 18 | 53 |
| SOD1 chr21:31851972+ | 1 | 2 | 3 | 3 | 5 | 52 |
| SOD1 chr21:31851992+ | 1 | 2 | 3 | 4 | 5 | 53 |
| SOD1 chr21:31852011+ | 2 | 4 | 4 | 5 | 7 | 78 |
| SOD1 chr21:31852013+ | 0 | 0 | 2 | 3 | 6 | 78 |
| SOD1 chr21:31852016+ | 0 | 3 | 4 | 5 | 8 | 78 |
| SOD1 chr21:31852026+ | 5 | 6 | 8 | 10 | 13 | 76 |
| SOD1 chr21:31852040+ | 4 | 5 | 6 | 7 | 9 | 76 |
| SOD1 chr21:31852045+ | 2 | 4 | 4 | 6 | 8 | 76 |
| SOD1 chr21:31852048+ | 2 | 3 | 4 | 5 | 7 | 71 |
| SOD1 chr21:31852053+ | 3 | 5 | 6 | 8 | 11 | 75 |
| SOD1 chr21:31852060+ | 0 | 4 | 5 | 7 | 9 | 75 |
| LPL chr8:19840674+ | 8 | 10 | 11 | 12 | 14 | 74 |
| **LPL chr8:19840701+** | **30** | **33** | **36** | **39** | **43** | 75 |
| **PPARg2 chr3:12367795+** | **59** | **70** | **73** | **76** | **80** | 68 |
| **PPARg2 chr3:12367792+** | **66** | **76** | **81** | **84** | **86** | 68 |
| **PPARg2 chr3:12367759+** | **53** | **58** | **61** | **64** | **66** | 68 |

**Supplementary Table S5: Distributions of CpGs measured by Sequenom (highlighted rows 75^{th} centile ≥5% and 5^{th}-95^{th} centiles ≥10%)**

| | **5th centile** | **25th centile** | **Median** | **75th centile** | **95th centile** | **No. subjects** |
|---|---|---|---|---|---|---|
| PIK3CD chr1:9609690+ | 0 | 1 | 2 | 3 | 13 | 56 |
| PIK3CD chr1:9609713+ | 0 | 0 | 0 | 0 | 7 | 57 |
| PIK3CDchr1:9609727+ | 0 | 0 | 0 | 0 | 0 | 55 |
| PIK3CDchr1:9609735,9609738+ | 0 | 0 | 0 | 1 | 5 | 58 |
| **PIK3CDchr1:9609747,9609749,9609752,9609755,9609758,9** | | | | | | 58 |
| **609762+** | **7** | **11** | **16** | **19** | **22** | |
| PIK3CDchr1:9609807+ | 2 | 3 | 3 | 4 | 10 | 58 |
| **PIK3CDchr1:9609870+** | **0** | **2** | **4** | **11** | **28** | 55 |
| PIK3CDchr1:9609885+ | 0 | 2 | 4 | 4 | 7 | 58 |
| PIK3CDchr1:9609900+ | 0 | 0 | 0 | 1 | 2 | 58 |
| **PIK3CDchr1:9609909,9609912,9609920+** | **1** | **3** | **4** | **5** | **19** | 58 |
| PIK3CDchr1:9609933+ | 0 | 1 | 1 | 2 | 2 | 58 |
| **PIK3CDchr1:9609980+** | **0** | **1** | **1** | **7** | **22** | 58 |
| PIK3CDchr1:9635355+ | 1 | 2 | 2 | 4 | 67 | 36 |
| PIK3CDchr1:9635375+ | 0 | 0 | 0 | 0 | 3 | 37 |
| PIK3CDchr1:9635385+ | 0 | 0 | 0 | 0 | 15 | 37 |
| PIK3CDchr1:9635393+ | 0 | 0 | 0 | 0 | 14 | 37 |
| PIK3CDchr1:9635409+ | 0 | 0 | 0 | 0 | 3 | 35 |
| PIK3CDchr1:9635433+ | 0 | 0 | 0 | 0 | 59 | 37 |
| **PIK3CDchr1:9635442,9635456+** | **2** | **4** | **5** | **8** | **22** | 37 |
| **PIK3CDchr1:9635515,9635587+** | **0** | **0** | **3** | **28** | **53** | 37 |
| **PIK3CDchr1:9635535,9635537+** | **1** | **3** | **5** | **9** | **43** | 37 |
| **PIK3CDchr1:9635676,9635679+** | **16** | **44** | **73** | **94** | **100** | 37 |
| **SOD1 chr21:31961522+** | **50** | **65** | **70** | **76** | **86** | 68 |
| **SOD1 chr21:31953263,31953483+** | **4** | **19** | **29** | **42** | **72** | 33 |
| **SOD1 chr21:31953394+** | **15** | **71** | **93** | **97** | **100** | 31 |
| **SOD1 chr21:31953511+** | **0** | **2** | **4** | **25** | **70** | 33 |
| **SOD1 chr21:31953606,31953608+** | **0** | **4** | **5** | **6** | **47** | 33 |
| **SOD1 chr21:31953619,31953622+** | **0** | **4** | **6** | **9** | **13** | 33 |
| SOD1 chr21:31953629,31953635+ | 2 | 3 | 4 | 6 | 8 | 33 |
| SOD1 chr21:31954426,31954428+ | 0 | 0 | 1 | 2 | 4 | 46 |
| SOD1 chr21:31954442+ | 0 | 0 | 0 | 0 | 2 | 46 |
| SOD1 chr21:31954461+ | 0 | 1 | 1 | 2 | 7 | 46 |
| SOD1 chr21:31954470+ | 0 | 0 | 0 | 0 | 2 | 46 |
| SOD1 chr21:31853332+ | 0 | 0 | 0 | 3 | 10 | 31 |
| SOD1 chr21:31853358+ | 0 | 0 | 2 | 4 | 5 | 30 |
| SOD1 chr21:31853393+ | 0 | 0 | 0 | 2 | 4 | 28 |
| SOD1 chr21:31853398+ | 0 | 0 | 0 | 0 | 3 | 31 |
| SOD1 chr21:31853487,31853489,31853491+ | 0 | 1 | 1 | 3 | 9 | 31 |
| **SOD1 chr21:31853537+** | **0** | **4** | **6** | **8** | **12** | 31 |
| SOD1 chr21:31853545+ | 0 | 0 | 2 | 4 | 7 | 31 |
| **SOD1 chr21:31853586,31853588,31853591,31853596+** | **11** | **18** | **25** | **27** | **34** | 31 |
| SOD1 chr21:31853641+ | 0 | 0 | 1 | 3 | 5 | 31 |
| SOD1 chr21:31853641+ | 2 | 4 | 4 | 5 | 6 | 67 |
| **SOD1 chr21:31853660,31853663+** | **2** | **5** | **7** | **8** | **13** | 66 |
| SOD1 chr21:31853692+ | 1 | 4 | 6 | 7 | 8 | 67 |
| SOD1 chr21:31853762+ | 0 | 1 | 1 | 2 | 3 | 67 |
| SOD1 chr21:31853828+ | 0 | 0 | 0 | 2 | 3 | 67 |
| **SOD1 chr21:31853837+** | **0** | **2** | **5** | **6** | **11** | 65 |
| SOD1 chr21:31853878,31853880+ | 3 | 4 | 5 | 5 | 10 | 67 |
| SOD1 chr21:31853887+ | 2 | 3 | 4 | 5 | 6 | 67 |
| **RXRA chr9:136355556,136355560+** | **4** | **30** | **50** | **85** | **100** | 63 |
| **RXRA chr9:136355569+** | **0** | **2** | **12** | **29** | **81** | 63 |
| **RXRA chr9:136355593,136355600+** | **5** | **27** | **50** | **75** | **100** | 62 |
| **RXRA chr9:136355688+** | **9** | **41** | **62** | **92** | **99** | 64 |
| **RXRA chr9:136355836+** | **3** | **21** | **50** | **82** | **99** | 64 |
| **RXRA chr9:136355885+** | **4** | **36** | **59** | **84** | **99** | 64 |
| **RXRA chr9:136357082,136357085,136357087+** | **4** | **6** | **8** | **12** | **17** | 63 |
| RXRA chr9:136357160,136357165+ | 0 | 2 | 3 | 5 | 6 | 63 |
| RXRA chr9:136357175+ | 2 | 3 | 5 | 6 | 8 | 63 |
| **RXRA chr9:136357196+** | **0** | **4** | **5** | **7** | **13** | 59 |
| RXRA chr9:136357211+ | 0 | 0 | 0 | 1 | 2 | 62 |
| **eNOS chr7:150315553+** | **64** | **83** | **93** | **97** | **100** | 66 |
| **eNOS chr7:150315604+** | **56** | **75** | **86** | **95** | **100** | 66 |
| IL8 chr4:74705034+ | 0 | 0 | 0 | 0 | 15 | 46 |
| **IL8 chr4:74705041,74705043,74705045,74705049+** | **6** | **9** | **11** | **20** | **54** | 45 |
| **IL8 chr4:74705094+** | **1** | **3** | **4** | **5** | **49** | 46 |
| IL8 chr4:74705109+ | 0 | 1 | 1 | 2 | 5 | 46 |
| **IL8 chr4:74705162+** | **3** | **6** | **8** | **12** | **35** | 46 |

**Supplementary Table S6: Characteristics of the subjects studied for Example 1 b**

| | **Median** (inter-quartile range) | **No**. **subjects** |
|---|---|---|
| **Princess Anne Hospital cohort** | | |
| *Birth weight, g* | *3330 (3010 - 3790)* | 78 |
| *Age at follow up, years* | *8.67 (8.52 - 8.73)* | 78 |
| Adiposity at follow up measured by dual-energy X-ray absorptiometry | | |
| Total fat mass, kg | 5.41 (3.97 - 8.41) | 78 |
| % body fat | 18.3 (14.4 - 27.0) | 78 |
| | | |

| **Southampton Women's Survey cohort** | | |
|---|---|---|
| *Birth weight, g* | *3420 (3080 - 3870)* | 27 |
| *Age at follow up, years* | *6.53 (6.41 - 6.69)* | *27* |
| Adiposity at follow up measured by dual-energy X-ray absorptiometry | | |
| Total fat mass, kg | 4.97 (3.65 - 6.30) | 27 |
| % body fat | 24.4 (20.3 - 29.0) | 27 |

| | | |
|---|---|---|
| Adiposity measures adjusted for sex and age | | |

**Supplementary Table S7: CpGs with correlations with dual energy X-ray absorptiometry (DXA) measurements of adiposity in PAH subjects**

| | | **DXA total fat mass** | **DXA %body fat** | **DXA ratio of trunk/limb fat** |
|---|---|---|---|---|
| SOD1 chr21:31953511+ | rₚ | -0.15 | -0.12 | -0.38 |
| | P value | 0.41 | 0.519 | 0.028* |
| | *No.* | *33* | *33* | *33* |
| SOD1 chr21;31953619/22+ | rₚ | 0.20 | 0.20 | 0.41 |
| | P value | 0.27 | 0.27 | 0.017* |
| | *No.* | *33* | *33* | *33* |
| SOD1 chr21:31853660/63+ | rₚ | -0.06 | -0.12 | -0.32 |
| | P value | 0.62 | 0.34 | 0.008* |
| | *No.* | *66* | *66* | *66* |
| RXRA chr9:136355556/60+ | rp | 0.24 | 0.26 | 0.11 |
| | P value | 0.061 | 0.043* | 0.39 |
| | *No.* | *63* | *63* | *63* |
| RXRA chr9:136355836+ | rₚ | 0.33 | 0.30 | 0.12 |
| | P value | 0.008* | 0.017* | 0.37 |
| | *No.* | *64* | *64* | *64* |
| RXRA chr9:136355885+ | rₚ | 0.32 | 0.29 | 0.26 |
| | P value | 0.009* | 0.023* | 0.039* |
| | *No.* | *64* | *64* | *64* |
| eNOS chr7:150315553+ | rₚ | 0.42 | 0.37 | 0.33 |
| | P value | <0.001* | 0.002* | 0.007* |
| | *No.* | 66 | *66* | *66* |

| | | | | |
|---|---|---|---|---|
| All analyses adjusted for the child's sex;.rp =Pearson correlation coefficient; * P<0.05 | | | | |

## Claims

1. A method of predicting future development of one or more indices of obesity or adiposity selected from such indices including total or proportionate body fat, body mass index and trunk/ limb fat ratio as an indicator of body fat distribution in a human,
said method comprising
(a) determining in genomic DNA of said human extracted from a tissue sample the methylation status of one or more of the following CpG dinucleotides and /or CpG groups 5' to the coding region of the retinoid X receptor-alpha (RXRA) gene as identified by genomic coordinates according to the UCSC human genome March 2006 assembly
| |
|---|
| RXRA chr9:136355885+ |
| RXRA chr9:136355556,136355560+ |
| RXRA chr9:136355836+ |
(b) wherein the methylation status of each selected CpG or CpG group correlates with propensity for one or more indices of obesity or adiposity selected from such indices including total or proportionate body fat, body mass index and trunk/ limb fat ratio as an indicator of body fat distribution and
(c) comparing each methylation status determined as in (a) with correlation between methylation status of the relevant CpG or CpG group and variance of one or more indices of obesity or adiposity selected from such indices including total or proportionate body fat, body mass index and trunk/ limb fat ratio as an indicator of body fat distribution in individuals of the same species at a selected older age

2. A method as claimed in claim 1 wherein said tissue sample is umbilical cord or another perinatal tissue sample.

3. A method as claimed in any one of claims 1 or 2 wherein said comparing step
(b) takes account of gender.

4. A method as claimed in any one of the preceding claims, wherein in step (c) the following correlations is relied on with reference to variance of one or more indices of obesity or adiposity selected from such indices including total or proportionate body fat, body mass index and trunk/ limb fat ratio as an indicator of body fat distribution in humans at an age post-infancy:
(a) positive correlation between the methylation status of RXRA chr9:136355885+ and one or more of total body fat mass, percentage body fat mass, trunk/ limb fat ratio and body mass index adjusted for sex.

5. A method as claimed in any one of the preceding claims wherein the methylation status of at least two CpG sites are determined 5' to the coding region of different genes in different mechanistic pathways to predict development of one or more indices of obesity or adiposity selected from such indices including total or proportionate body fat, body mass index and trunk/ limb fat ratio as an indicator of body fat distribution.

6. A kit for carrying out a method a claimed in any one of the preceding claims comprising: (i) primers having the sequences represented by SEQ ID NOs: 30 and 31, for obtaining amplicons to analyse the methylation status of said one or more selected CpG dinucleotides and /or CpG groups, optionally together with means for bisulphite treatment of DNA and (ii) information on correlation between each said methylation status and variance of a phenotypic characteristic as required for comparing step (b).

## Patentansprüche

1. Verfahren zum Vorhersagen der zukünftigen Entwicklung von einem oder mehreren Indizes von Fettleibigkeit oder Adipositas, ausgewählt aus derartigen Indizes einschließlich gesamtem oder anteilmäßigem Körperfett, Body-Mass-Index und Rumpf/Gliedmaßen-Fett-Verhältnis, als Indikator der Körperfettverteilung bei einem Menschen,
wobei das Verfahren umfasst
(a) Bestimmen in genomischer DNA des Menschen, extrahiert aus einer Gewebeprobe, des Methylierungsstatus von einem oder mehreren von den folgenden CpG-Dinucleotiden und/oder CpG-Gruppen 5' zu der Codierungsregion des Retinoid-X-Rezeptor-alpha-(RXRA)-Gens, wie identifiziert durch genomische Koordinaten entsprechend dem UCSC human genome March 2006 assembly (Aufbau des menschlichen Genoms, UCSC, März 2006)
RXRA chr9:136355885+
RXRA chr9:136355556, 136355560+
RXRA chr9:136355836+
(b) wobei der Methylierungsstatus von jedem ausgewählten CpG oder jeder CpG-Gruppe mit Anfälligkeit für einen oder mehrere Indizes von Fettleibigkeit oder Adipositas, ausgewählt aus derartigen Indizes einschließlich gesamtem oder anteilmäßigem Körperfett, Body-Mass-Index und Rumpf/Gliedmaßen-Fett-Verhältnis, als Indikator der Körperfettverteilung korreliert und
(c) Vergleichen von jedem Methylierungsstatus, bestimmt wie in (a), mit Korrelation zwischen Methylierungsstatus des relevanten CpG oder der CpG-Gruppe und Varianz von einem oder mehreren Indizes von Fettleibigkeit oder Adipositas, ausgewählt aus derartigen Indizes einschließlich gesamtem oder anteilmäßigem Körperfett, Body-Mass-Index und Rumpf/Gliedmaßen-Fett-Verhältnis, als Indikator der Körperfettverteilung in Individuen der gleichen Spezies mit einem ausgewählten höheren Alter.

2. Verfahren, wie in Anspruch 1 beansprucht, wobei die Gewebeprobe die Nabelschnur oder eine andere perinatale Gewebeprobe ist.

3. Verfahren, wie in einem der Ansprüche 1 oder 2 beansprucht, wobei der Vergleichsschritt das Geschlecht berücksichtigt.

4. Verfahren, wie in einem der vorhergehenden Ansprüche beansprucht, wobei in Schritt (c) die folgenden Korrelationen sich auf Bezugnahme auf Varianz von einem oder mehreren Indizes von Fettleibigkeit oder Adipositas, ausgewählt aus derartigen Indizes einschließlich gesamtem oder anteilmäßigem Körperfett, Body-Mass-Index und Rumpf/Gliedmaßen-Fett-Verhältnis, als Indikator der Körperfettverteilung bei Menschen in einem Alter nach der frühen Kindheit stützen:
(a) positive Korrelation zwischen dem Methylierungsstatus von RXRA chr9:136355885+ und einem oder mehreren von gesamter Körperfettmasse, prozentualer Körperfettmasse, Rumpf/Gliedmaßen-Fett-Verhältnis und Body-Mass-Index, angepasst für das Geschlecht.

5. Verfahren, wie in einem der vorhergehenden Ansprüche beansprucht, wobei der Methylierungsstatus von mindestens zwei CpG-Stellen 5' zu der Codierungsregion von unterschiedlichen Genen in unterschiedlichen mechanistischen Wegen bestimmt wird, um Entwicklung von einem oder mehreren Indizes von Fettleibigkeit oder Adipositas, ausgewählt aus derartigen Indizes einschließlich gesamtem oder anteilmäßigem Körperfett, Body-Mass-Index und Rumpf/Gliedmaßen-Fett-Verhältnis als Indikator der Körperfettverteilung, vorherzusagen.

6. Kit zum Ausführen eines Verfahrens, wie in einem der vorhergehenden Ansprüche beansprucht, umfassend: (i) Primer, aufweisend die Sequenzen, dargestellt durch die SEQ ID NOs: 30 und 31, zum Gewinnen von Amplicons, um den Methylierungsstatus von dem einen oder den mehreren ausgewählten CpG-Dinucleotiden und/oder CpG-Gruppen zu analysieren, gegebenenfalls zusammen mit Mitteln zur Bisulfit-Behandlung von DNA, und (ii) Information zur Korrelation zwischen jedem von dem Methylierungsstatus und der Varianz einer phänotypischen charakteristischen Eigenschaft, wie erforderlich für Vergleichsschritt (b).

## Revendications

1. Méthode pour la prédiction du développement futur d'un ou de plusieurs indices d'obésité ou d'adiposité choisis parmi de tels indices incluant la graisse corporelle totale ou proportionnée, l'indice de masse corporelle et le rapport de graisse tronc/membre comme indicateur de la distribution de graisse corporelle chez un humain,
ladite méthode comprenant:
(a) la détermination dans un ADN génomique dudit humain extrait à partir d'un échantillon de tissu de l'état de méthylation d'un ou de plusieurs des dinucléotides CpG qui suivent et/ou des groupes de CpG en 5' par rapport à la région de codage du gène de récepteur alpha X rétinoïde (RXRA) tel qu'identifié par les coordonnées génomiques selon l'assemblage de génomes humains UCSG de mars 2006
RXRA chr9: 136355885+
RXRA chr9: 136355556, 136355560+
RXRA chr9: 136355836+
(b) dans laquelle l'état de méthylation de chaque CpG ou groupe CpG sélectionné est en corrélation avec la propension pour un ou plusieurs indices d'obésité ou d'adiposité choisis parmi de tels indices incluant la graisse corporelle totale ou proportionnée, l'indice de masse corporelle et le rapport de graisse tronc/membre comme indicateur de la distribution de graisse corporelle et
(c) la comparaison de chaque état de méthylation déterminé comme dans (a) avec la corrélation entre l'état de méthylation du CpG ou du groupe CpG pertinent et la variance d'un ou de plusieurs indices d'obésité ou d'adiposité choisis parmi de tels indices incluant la graisse corporelle totale ou proportionnée, l'indice de masse corporelle et le rapport de graisse tronc/membre comme indicateur de la distribution de graisse corporelle chez des individus de la même espèce à un âge plus vieux sélectionné.

2. Méthode selon la revendication 1, dans laquelle ledit échantillon de tissu est un cordon ombilical ou un autre échantillon de tissu périnatal.

3. Méthode selon l'une quelconque des revendications 1 ou 2, dans laquelle ladite étape de comparaison (b) tient compte du genre.

4. Méthode selon l'une quelconque des revendications précédentes, dans laquelle dans l'étape (c) la corrélation qui suit repose sur la référence à la variance d'un ou de plusieurs indices d'obésité ou d'adiposité choisis parmi de tels indices incluant la graisse corporelle totale ou proportionnée, l'indice de masse corporelle et le rapport de graisse tronc/membre comme indicateur de la distribution de graisse corporelle chez des humains à un âge après l'enfance:
(a) corrélation positive entre l'état de méthylation de RXRA chr9: 136355885+ et un ou plusieurs parmi la masse de graisse totale, le pourcentage de masse de graisse corporelle, le rapport de graisse tronc/membre et l'indice de masse corporelle ajustés au sexe.

5. Méthode selon l'une quelconque des revendications précédentes, dans laquelle les états de méthylation d'au moins deux sites CpG sont déterminés en 5' par rapport à la région de codage de différents gènes dans différentes voies mécanistiques pour prédire le développement d'un ou de plusieurs indices d'obésité ou d'adiposité choisis parmi de tels indices incluant la graisse corporelle totale ou proportionnée, l'indice de masse corporelle et le rapport de graisse tronc/membre comme indicateur de la distribution de graisse corporelle.

6. Kit pour réaliser une méthode selon l'une quelconque des revendications précédentes comprenant: (i) des amorces possédant les séquences représentées par les SEQ ID NOs: 30 et 31, pour l'obtention d'amplicons pour analyser l'état de méthylation desdits un ou plusieurs dinucléotides CpG et/ou groupes de CpG sélectionnés, optionnellement accompagnées d'un moyen pour un traitement au bisulfite d'ADN et (ii) une information sur la corrélation entre chacun desdits états de méthylations et la variance d'une caractéristique phénotypique telle que requise pour l'étape de comparaison (b).
